# EUROPEAN PATENT SPECIFICATION

(11) **EP 1 686 113 B1**
(45) Date of publication and mention of the grant of the patent: **23.11.2011**
(21) Application number: 06009072.7
(22) Date of filing: 08.06.2000
(51) Int. Cl.: C07C 311/16, C07C 311/29, C07D 317/46, A61K 31/36, A61P 31/18, A61K 31/352, C07D 493/04

(54) **Inhibitors of aspartyl protease**
Aspartylprotease-Inhibitoren
Inhibiteurs d'aspartyl protease

(30) Priority: 11.06.1999 US 139070 P; 17.03.2000 US 190211 P
(43) Date of publication of application: 02.08.2006
(62) Divisional of application: 00941279.2
(73) Proprietor: VERTEX PHARMACEUTICALS INCORPORATED, Cambridge, MA 02139-4242 (US)
(72) Inventor: Hale, Michael, Robin, Bedford, MA 01730 (US); Tung, Roger, San Diego, CA 92130 (US); Price, Stephen, Aylesbury, Buchs, HP225UJ (GB); Wilkes, Robin David, Didcot, Oxon OX11 7UL (GB); Schairer, Wayne, Carl, Westborough, MA 01581 (US); Jarvis, Ashley Nicholas, Osney, Oxford OX2 OBH (GB); Spaltenstein, Andrew, Raleigh, NC 27606 (US); Furfine, Eric Steven, Durham, NC 27707 (US); Samano, Vicente, Chapel Hill, NC 27514 (US); Kaldor, Istvan, Durham, NC 27703 (US); Miller, John, Franklin, Durham, NC 27712 (US); Brieger, Michael Stephen, Durham NC 27712 (US)
(74) Representative: Vossius & Partner

(56) References cited:
- EP-A- 0 434 365
- WO-A-94/10136
- US-A- 5 622 949
- US-A- 5 744 481
- US-A- 5 843 946
- GHOSH A K ET AL: "Potent HIV protease inhibitors incorporating high-affinity P2-ligands and (R)-(hydroxyethylamino)sulfonamide isostere" BIOORGANIC & MEDICINAL CHEMISTRY LETTERS,GB,OXFORD, vol. 8, no. 6, 17 March 1998 (1998-03-17), pages 687-690, XP004136945 ISSN: 0960-894X
- FRESKOS J N ET AL: "(Hydroxyethyl) Sulfonamide HIV-1 Protease Inhibitors: Identification Of The 2-Methylbenzoyl Moiety At P-2" BIOORGANIC & MEDICINAL CHEMISTRY LETTERS,GB,OXFORD, vol. 6, no. 4, 20 February 1996 (1996-02-20), pages 445-450, XP004135054 ISSN: 0960-894X

## Description

### TECHNICAL FIELD OF THE INVENTION

The present invention relates to a novel class of sulfonamides which are aspartyl protease inhibitors. In one embodiment, this invention relates to a novel class of HIV aspartyl protease inhibitors characterized by specific structural and physicochemical features. This invention also relates to pharmaceutical compositions comprising these compounds. The compounds and pharmaceutical compositions of this invention are particularly well suited for inhibiting HIV-1 and HIV-2 protease activity and consequently, may be advantageously used as anti-viral agents against the HIV-1 and HIV-2 viruses.

### BACKGROUND OF THE INVENTION

The human immunodeficiency virus ("HIV") is the causative agent for acquired immunodeficiency syndrome ("AIDS") -- a disease characterized by the destruction of the immune system, particularly of CD4⁺ T-cells, with attendant susceptibility to opportunistic infectionsand its precursor AIDS-related complex ("ARC") -- a syndrome characterized by symptoms such as persistent generalized lymphadenopathy, fever and weight loss.

As in the case of several other retroviruses, HIV encodes the production of a protease which carries out post-translational cleavage of precursor polypeptides in a process necessary for the formation of infectious virions (S. Crawford et al., "A Deletion Mutation in the 5' Part of the pol Gene of Moloney Murine Leukemia Virus Blocks Proteolytic Processing of the gag and pol Polyproteins", J. Virol., 53, p. 899 (1985)). These gene products include pol, which encodes the virion RNA-dependent DNA polymerase (reverse transcriptase), an endonuclease, HIV protease, and gag, which encodes the core-proteins of the virion (H. Toh et al., "Close Structural Resemblance Between Putative Polymerase of a Drosophila Transposable Genetic Element 17.6 and pol gene product of Moloney Murine Leukemia Virus", EMBO J., 4, p. 1267 (1985); L.H. Pearl et al., "A Structural Model for the Retroviral Proteases", Nature, pp. 329-351 (1987); M.D. Power et al., "Nucleotide Sequence of SRV-1, a Type D Simian Acquired Immune Deficiency Syndrome Retrovirus", Science, 231, p. 1567 (1986)).

A number of synthetic anti-viral agents have been designed to target various stages in the replication cycle of HIV. These agents include compounds which block viral binding to CD4⁺ T-lymphocytes (for example, soluble CD4), and compounds which interfere with viral replication by inhibiting viral reverse transcriptase (for example, didanosine and zidovudine (AZT)) and inhibit integration of viral DNA into cellular DNA (M.S. Hirsh and R.T. D'Aqulia, "Therapy for Human Immunodeficiency Virus Infection", N.Eng.J.Med., 328, p. 1686 (1993)). However, such agents, which are directed primarily to early stages of viral replication, do not prevent the production of infectious virions in chronically infected cells. Furthermore, administration of some of these agents in effective amounts has led to cell-toxicity and unwanted side effects, such as anemia and bone marrow suppression.

More recently, the focus of anti-viral drug design has been to create compounds which inhibit the formation of infectious virions by interfering with the processing of viral polyprotein precursors. Processing of these precursor proteins requires the action of virus-encoded proteases which are essential for replication (Kohl., N.E. et al. "Active HIV Protease is Required for Viral Infectivity" Proc. Natl. Acad. Sci. USA, 85, p. 4686 (1988)). The anti-viral potential of HIV protease inhibition has been demonstrated using peptidyl inhibitors.

More recently several small molecule protease inhibitors have become available for the treatment of HIV infections. Among these is the sulfonamide-containing molecule, Agenerase^{®} (amprenavir). Agenerase^{®} is described in United States patent 5,585,397. Other sulfonamide inhibitors of aspartyl protease are described in United States patents 5,691,372, 5,510,388, 5,521,219; 5,639,769, 5,714,605, 5,744,481, 5,786,483, 5,830,897 and 5,843,946.

Because HIV-infected patients often develop resistance to particular protease inhibitors, the need still exists for additional compounds that can effectively inhibit the action of aspartyl proteases, particularly HIV protease, for use as agents for preventing and treating chronic and acute viral infections. Moreover, there is also a need for compounds that can effectively inhibit the action of HIV mutants which are resistant to conventional protease inhibitors.

### SUMMARY OF THE INVENTION

The present invention provides a novel class of compounds, and pharmaceutically acceptable derivatives thereof, that are useful as inhibitors of aspartyl proteases, in particular, HIV aspartyl protease. These compounds can be used alone or in combination with other therapeutic or prophylactic agents, such as anti-virals, antibiotics, immunomodulators or vaccines, for the treatment or prophylaxis of viral infection.

According to a preferred embodiment, the compounds of this invention are capable of inhibiting HIV viral replication in human CD₄⁺ T-cells. These compounds are useful as therapeutic and prophylactic agents to treat or prevent infection by HIV-1 and related viruses which may result in asymptomatic infection, AIDS-related complex ("ARC"), acquired immunodeficiency syndrome ("AIDS"), or similar disease of the immune system.

According to another preferred embodiment, the compounds of this invention are useful as therapeutic and prophylactic agents to treat or prevent infection by HIV mutants.

It is a principal object of this invention to provide a novel class of sulfonamides which are aspartyl protease inhibitors, and particularly, HIV aspartyl protease inhibitors. The novel sulfonamides of this invention are those as cited in claim 1.

It is also an object of this invention to provide pharmaceutical compositions comprising the sulfonamides of formula (I) and their use as inhibitors of HIV aspartyl protease.

### DETAILED DESCRIPTION OF THE INVENTION

In order that the invention herein described may be more fully understood, the following detailed description is set forth. In the description, the following terms are employed herein:
Unless expressly stated to the contrary, the terms "-SO₂-" and "-S (O)₂-" as used herein refer to a sulfone or sulfone derivative (i.e., both appended groups linked to the S), and not a sulfinate ester.

For the compounds of formula I, and intermediates thereof, the stereochemistry of OR⁷ is defined relative to D on the adjacent carbon atom, when the molecule is drawn in an extended zigzag representation (such as that drawn for compound of formula I). If both OR⁷ and D reside on the same side of the plane defined by the extended backbone of the compound, the stereochemistry of OR⁷ will be referred to as "syn". If OR⁷ and D reside on opposite sides of that plane, the stereochemistry of OR⁷ will be referred to as "anti".

The term "pharmaceutically effective amount" refers to an amount effective in treating a virus infection, for example an HIV infection, in a patient either as monotherapy or in combination with other agents. The term "treating" as used herein refers to the alleviation of symptoms of a particular disorder in a patient or the improvement of an ascertainable measurement associated with a particular disorder. The term "prophylactically effective amount" refers to an amount effective in preventing a virus infection, for example an HIV infection, in a patient. As used herein, the term "patient" refers to a mammal, including a human.

The terms "HIV protease" and "HIV aspartyl protease" are used interchangeably and refer to the aspartyl protease encoded by the human immunodeficiency virus type 1 or 2. In a preferred embodiment of this invention, these terms refer to the human immunodeficiency virus type 1 aspartyl protease.

The term "thiocarbamates" refers to compounds containing the functional group N-SO₂-O.

Combinations of substituents and variables envisioned by this invention are only those that result in the formation of stable compounds. The term "stable", as used herein, refers to compounds which possess stability sufficient to allow manufacture and administration to a mammal by methods known in the art. Typically, such compounds are stable at a temperature of 40°C or less, in the absence of moisture or other chemically reactive conditions, for at least a week.

This invention also envisions the quaternization of any basic nitrogen-containing groups of the compounds disclosed herein. The basic nitrogen can be quaternized with any agents known to those of ordinary skill in the art including, for example, lower alkyl halides, such as methyl, ethyl, propyl and butyl chloride, bromides and iodides; dialkyl sulfates including dimethyl, diethyl, dibutyl and diamyl sulfates; long chain halides such as decyl, lauryl, myristyl and stearyl chlorides, bromides and iodides; and aralkyl halides including benzyl and phenethyl bromides. Water or oil-soluble or dispersible products may be obtained by such quaternization. The novel sulfonamides of this invention are those claimed in claim 1.
and pharmaceutically acceptable salts thereof; wherein:

It will be understood by those of skill in the art that component M or M' in the formulae set forth herein will have either a covalent, a covalent/ zwitterionic, or an ionic association with either Z or R⁹ depending Upon the actual choice for M or M'. When M or M' is hydrogen, alkyl, alkenyl, or R⁶, M or M' is covalently bound to R⁹ or Z. If M is a mono- or bivalent metal or other charged species (i.e., NH₄⁺), there is an ionic interaction between M and Z and the resulting compound is a salt.

When x is 0 in (M)ₓ, Z may be a charged species. When that occurs, the other M may be oppositely charged to produce a 0 net charge on the molecule. Alternatively, the counter ion may located elsewhere in the molecule.

The compounds according to the invention contain one or more asymmetric carbon atoms and thus occur as racemates and racemic mixtures, single enantiomers, diastereomeric mixtures and individual diastereomers. All such isomeric forms of these compounds are expressly included in the present invention. Each stereogenic carbon may be of the R or S configuration. Although the specific compounds exemplified in this application may be depicted in a particular stereochemical configuration, compounds having either the opposite stereochemistry at any given chiral center or mixtures thereof are also envisioned.

The compounds of the present invention are set forth below in Tables 1, 2 and 3. The arrows in Tables 1 and 2, and the dotted lines in Table 3. indicate where the indicated moiety attaches to the rest of the molecule.

**Table 1:**

| | | | |
|---|---|---|---|
| 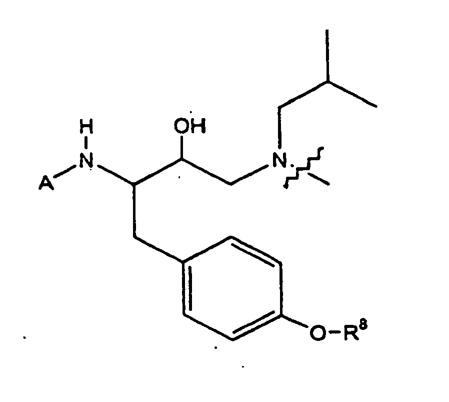 | | | |

| | A | 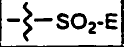 | R⁸ |
|---|---|---|---|
| 5a | 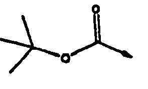 | 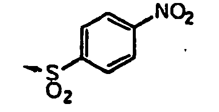 | 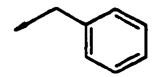 |
| 5b | 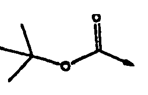 | 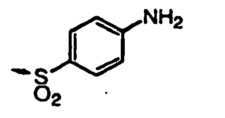 | 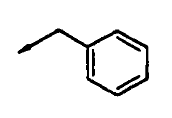 |
| 5c | 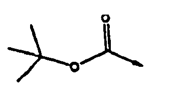 | 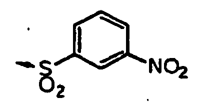 | 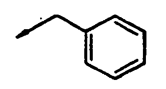 |
| 5d | 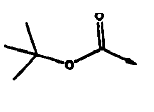 | 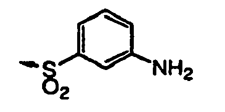 | 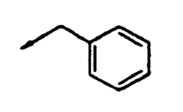 |
| 5e | 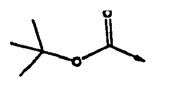 | 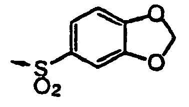 | 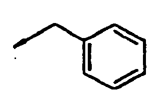 |
| 5f | 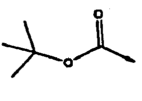 | 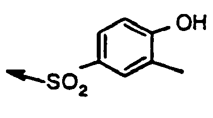 | 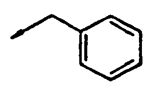 |
| 5g | 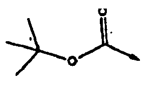 | 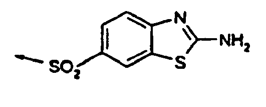 | 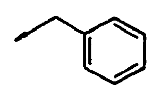 |
| 10 | 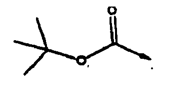 | 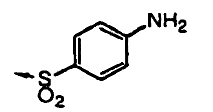 | 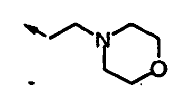 |
| 11 | 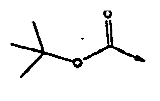 | 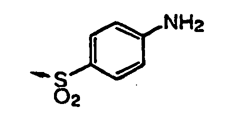 | 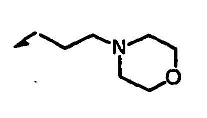 |
| 12 | 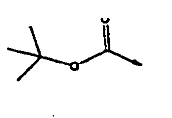 | 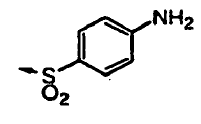 | 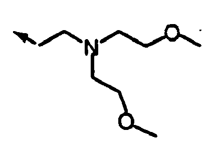 |
| 13 | 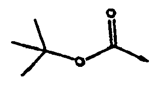 | 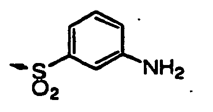 | 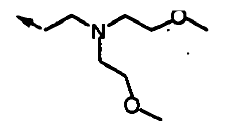 |
| 14 | 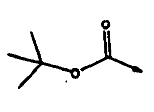 | 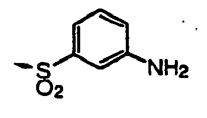 | 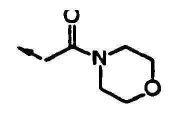 |
| 15 | 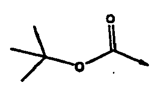 | 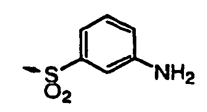 | 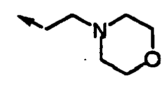 |
| 16 | 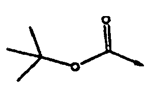 | 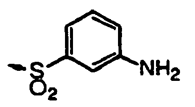 | 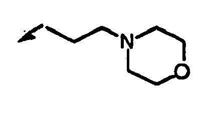 |
| 17 | 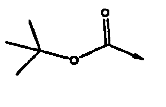 | 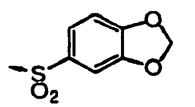 | 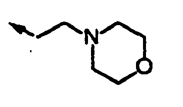 |
| 18 | 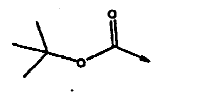 | 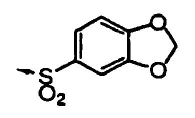 | 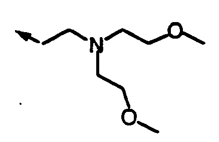 |
| 19 | 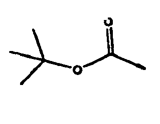 | 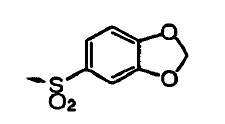 | 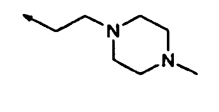 |
| 20 | 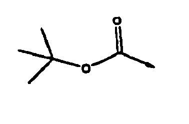 | 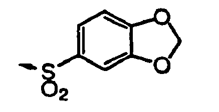 | 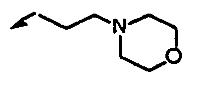 |
| 21 | 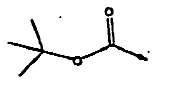 | 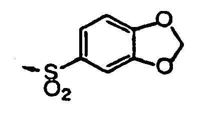 | 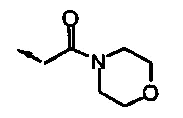 |
| 58 | 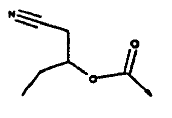 | 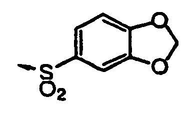 | 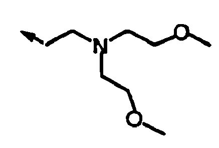 |

**Table 2:**

| | | | |
|---|---|---|---|
| 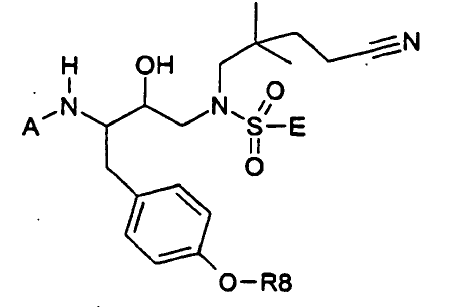 | | | |

| | A | S(O)₂E | R⁸ |
|---|---|---|---|
| 67 | 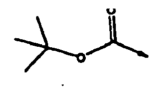 | 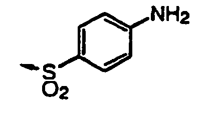 | 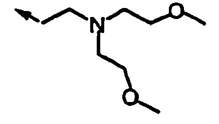 |
| 68 | 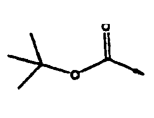 | 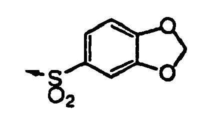 | 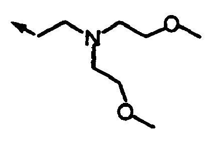 |

**Table 3**

| | | | | |
|---|---|---|---|---|
| 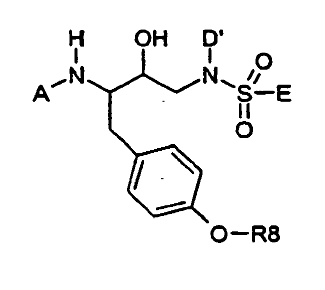 | | | | |

| | A | R⁸ | D' | E |
|---|---|---|---|---|
| 328 | 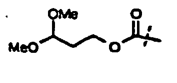 | 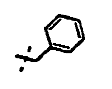 | 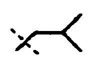 | 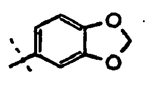 |

Preferred compound of the present invention are compound numbers: 18, 19, 20, 58 and 68.

More preferred is compound number : 58.

The compounds of the present invention can be readily prepared by techniques known in the art. Scheme I illustrates a general synthetic route to the compounds of this invention.

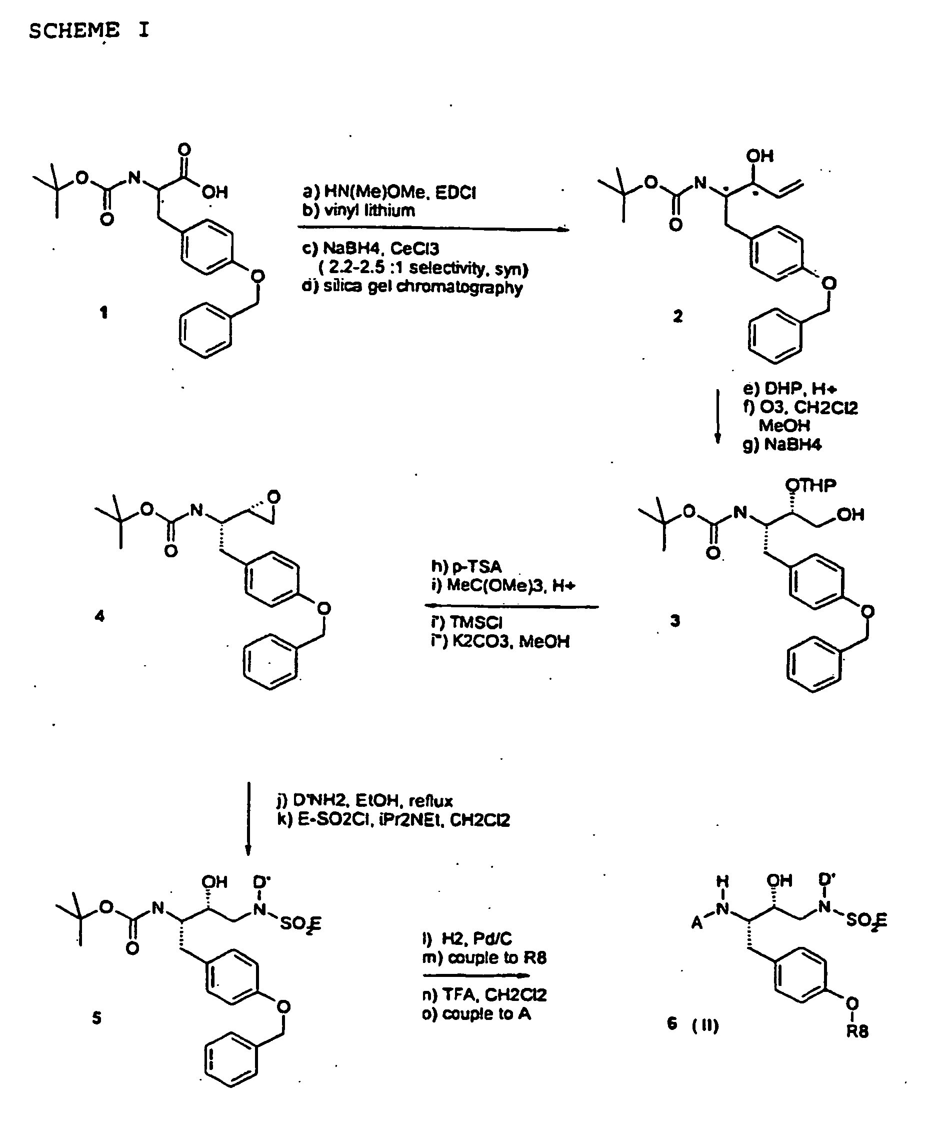

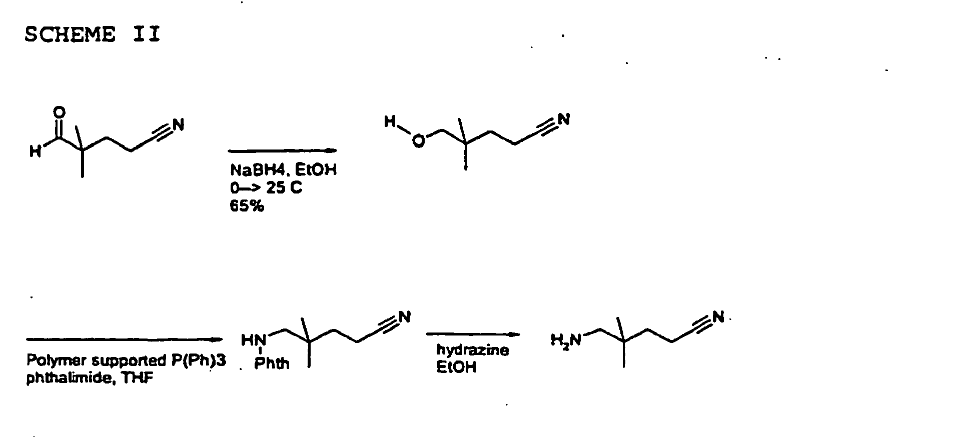

In Step 1 of Scheme I, the bis protected amino acid is homologated through initial conversion to the Wienreb Amide (a) followed by alkylation with vinyl lithium (b) and stereoselective reduction (c). The diastereomers can be seperated by silica gel chromatography (d). In step 2, the secondary alcohol is protected as a THP ether (e), as was found neccesary for the oxidation step. The olefin was then oxidized to the aldehyde by ozone and the resulting ozonide reduced to the alcohol by sodium borohydride (steps f and g). After removal of the THP group (h) under acidic conditions the diol was converted to the epoxide (i, i' and i") in one pot according to the method of Sharpless [K. B. Sharpless Tetrahedron 1992, 48 (35), pp. 10515-10530. The epoxide, 4, was then opened by H₂N-D' and further acylated in the presence of i-Pr₂Net by E-SO₂Cl to generate compounds of the formula schematically represented as 5. Alternate D' groups may be introduced at this point too. The synthesis of the D' as shown in compounds illustrated in Table II is shown in Scheme II.

These compounds could then be further manipulated by removal of the Bn group and introduction of a variety of R⁸ groups by reacting with the cooresponding alkyl halides. Further elaboration was possible by removal of the t-Butyl carbonate (1) and reintroduction of another group or carbamate designated as A, to provide compounds represented as 6 (formula II). We found that coupling as in reacton "m" was efficient under the following general conditions: alkyl halide (R⁸-Cl, 2.5 EQ. CsCO₃, dioxane, 80°C, 2-4 hours. Similar alkylating conditions are reported in J. Med. Chem 1992, 1688 along with representative routes for the synthesis of some R⁸-Cl intermediates. The coupling as illustrated for bringing in A, step "o", was generally efficient under the following conditions: activated p-NO₂-phenyl carbonate (p-NO₂-O-A), i-Pr₂NEt, CH₂Cl₂, RT, 12 hours. Use of the activated succinate provides an alternative coupling reagent (Succinate-A).

Alternatively, compounds of the present invention can also be prepared according to Scheme III below.

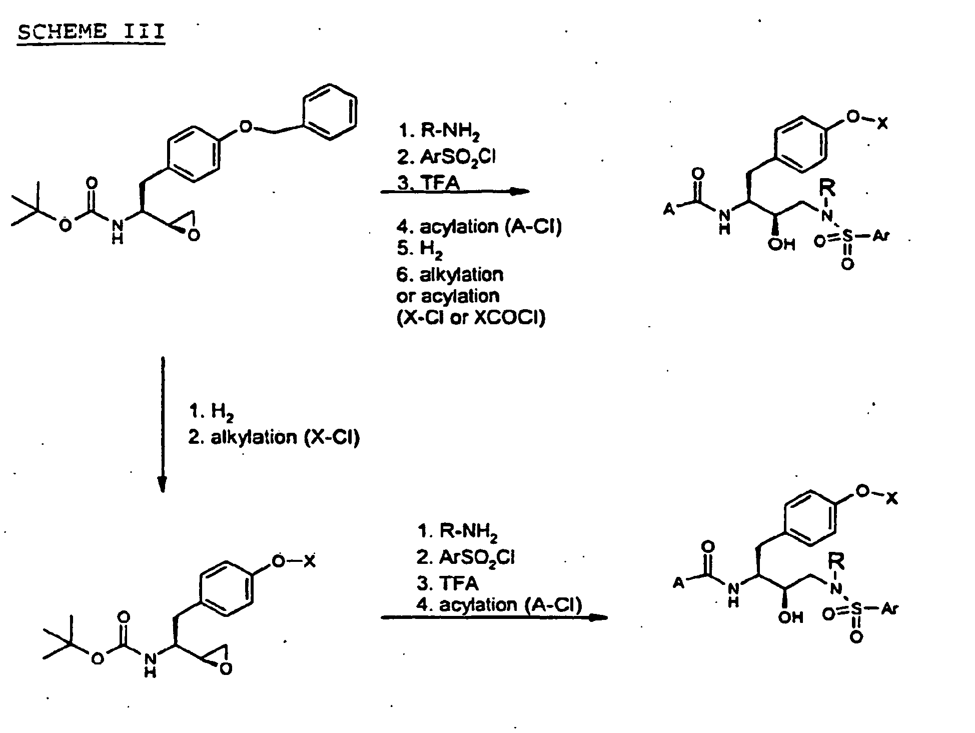

Thus, the synthetic approach illustrated in Scheme I and Scheme III can be readily extended to produce other compounds of the present invention. The above synthetic schemes are not intended to comprise a comprehensive list of all means by which compounds described and claimed in this application may be synthesized. Further methods will be evident to those of ordinary skill in the art.

As discussed above, the novel compounds of the present invention are excellent ligands for aspartyl proteases, particularly HIV-1 and HIV-2 proteases. Accordingly, these compounds are capable of targeting and inhibiting late stage events in HIV replication, i.e., the processing of the viral polyproteins by HIV encoded proteases. Such compounds inhibit the proteolytic processing of viral polyprotein precursors by inhibiting aspartyl protease. Because aspartyl protease is essential for the production of mature virions, inhibition of that processing effectively blocks the spread of virus by inhibiting the production of infectious virions, particularly from chronically infected cells. Compounds according to this invention advantageously inhibit the ability of the HIV-1 virus to infect immortalized Human T cells over a period of days, as determined by an assay of extracellular p24 antigena specific marker of viral replication. Other anti-viral assays have confirmed the potency of these compounds.

The compounds of this invention may be employed in a conventional manner for the treatment of viruses, such as HIV and HTLV, which depend on aspartyl proteases for obligatory events in their life cycle. Such methods of treatment, their dosage levels and requirements may be selected by those of ordinary skill in the art from available methods and techniques. For example, a compound of this invention may be combined with a pharmaceutically acceptable adjuvant for administration to a virally-infected patient in a pharmaceutically acceptable manner and in an amount effective to lessen the severity of the viral infection.

Alternatively, the compounds of this invention may be used in vaccines and methods for protecting individuals against viral infection over an extended period of time. The compounds may be employed in such vaccines either alone or together with other compounds of this invention in a manner consistent with the conventional utilization of protease inhibitors in vaccines. For example, a compound of this invention may be combined with pharmaceutically acceptable adjuvants conventionally employed in vaccines and administered in prophylactically effective amounts to protect individuals over an extended period time against HIV infection. As such, the novel protease inhibitors of this invention can be administered as agents for treating or preventing HIV infection in a mammal.

The compounds of the invention, especially those having a molecular weight of less than about 700 g/mole, may be readily absorbed by the bloodstream of mammals upon oral administration. Compounds of formula I having a molecular weight of less than about 600 g/mole are most likely to demonstrate oral availability. This surprisingly impressive oral availability makes such compounds excellent agents for orally-administered treatment and prevention regimens against HIV infection.

The compounds of this invention may be administered to a healthy or HIV-infected patient either as a single agent or in combination with other anti-viral agents which interfere with the replication cycle of HIV. By administering the compounds of this invention with other anti-viral agents which target different events in the viral life cycle, the therapeutic effect of these compounds is potentiated. For instance, the co-administered anti-viral agent can be one which targets early events in the life cycle of the virus, such as cell entry, reverse transcription and viral DNA integration into cellular DNA. Anti-HIV agents targeting such early life cycle events included, didanosine (ddI), alcitabine (ddC), d4T, zidovudine (AZT), polysulfated polysaccharides, sT4 (soluble CD4), ganiclovir, dideoxycytidine, trisodium phosphonoformate, eflornithine, ribavirin, acyclovir, alpha interferon and trimenotrexate. Additionally, non-nucleoside inhibitors of reverse transcriptase, such as TIBO or nevirapine, may be used to potentiate the effect of the compounds of this invention, as may viral uncoating inhibitors, inhibitors of trans-activating proteins such as tat or rev, or inhibitors of the viral integrase.

Combination therapies according to this invention exert a synergistic effect in inhibiting HIV replication because each component agent of the combination acts on a different site of HIV replication. The use of such combinations also advantageously reduces the dosage of a given conventional anti-retroviral agent which would be required for a desired therapeutic or prophylactic effect as compared to when that agent is administered as a monotherapy. These combinations may reduce or eliminate the side effects of conventional single anti-retroviral agent therapies while not interfering with the anti-retroviral activity of those agents. These combinations reduce potential of resistance to single agent therapies, while minimizing any associated toxicity. These combinations may also increase the efficacy of the conventional agent without increasing the associated toxicity. In particular, we have discovered that these compounds act synergistically in preventing the replication of HIV in human T cells. Preferred combination therapies include the administration of a compound of this invention with AZT, ddI, ddC or d4T.

Alternatively, the compounds of this invention may also be co-administered with other HIV protease inhibitors such as Ro 31-8959 (Roche), L-735,524 (Merck), XM 323 (Du-Pont Merck) and A-80,987 (Abbott) to increase the effect of therapy or prophylaxis against various viral mutants or members of other HIV quasi species.

We prefer administering the compounds of this invention as single agents or in combination with retroviral reverse transcriptase inhibitors, such as derivatives of AZT, or other HIV aspartyl protease inhibitors. We believe that the co-administration of the compounds of this invention with retroviral reverse transcriptase inhibitors or HIV aspartyl protease inhibitors may exert a substantial synergistic effect, thereby preventing, substantially reducing, or completely eliminating viral infectivity and its associated symptoms.

The compounds of this invention can also be administered in combination with immunomodulators (e.g., bropirimine, anti-human alpha interferon antibody, IL-2, GM-CSF, methionine enkephalin, interferon alpha, diethyldithiocarbamate, tumor necrosis factor, naltrexone and rEPO); and antibiotics (e.g., pentamidine isethiorate) to prevent or combat infection and disease associated with HIV infections, such as AIDS and ARC.

When the compounds of this invention are administered in combination therapies with other agents, they may be administered sequentially or concurrently to the patient. Alternatively, pharmaceutical or prophylactic compositions according to this invention may be comprised of a combination of an aspartyl protease inhibitor of this invention and another therapeutic or prophylactic agent.

Although this invention focuses on the use of the compounds disclosed herein for preventing and treating HIV infection, the compounds of this invention can also be used as inhibitory agents for other viruses which depend on similar aspartyl proteases for obligatory events in their life cycle. These viruses include, as well as other AIDS-like diseases caused by retroviruses, such as simian immunodeficiency viruses, but are not limited to, HTLV-I and HTLV-II. In addition, the compounds of this invention may also be used to inhibit other aspartyl proteases, and in particular, other human aspartyl proteases, including renin and aspartyl proteases that process endothelin precursors. Pharmaceutical compositions of this invention comprise any of the compounds of the present invention, and pharmaceutically acceptable salts thereof, with any pharmaceutically acceptable carrier, adjuvant or vehicle. Pharmaceutically acceptable carriers, adjuvants and vehicles that may be used in the pharmaceutical compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The pharmaceutical compositions of this invention may be administered orally, parenterally, by inhalation spray, topically, rectally, nasally, buccally, vaginally or via an implanted reservoir. We prefer oral administration or administration by injection. The pharmaceutical compositions of this invention may contain any conventional non-toxic pharmaceutically-acceptable carriers, adjuvants or vehicles. The term parenteral as used herein includes subcutaneous, intracutaneous, intravenous, intramuscular, intra-articular, intrasynovial, intrasternal, intrathecal, intralesional and intracranial injection or infusion techniques.

The pharmaceutical compositions may be in the form of a sterile injectable preparation, for example, as a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally-acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant such as Ph. Helv or a similar alcohol.

The pharmaceutical compositions of this invention may be orally administered in any orally acceptable dosage form including, but not limited to, capsules, tablets, and aqueous suspensions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are administered orally, the active ingredient is combined with emulsifying and suspending agents. If desired, certain sweetening and/or flavoring and/or coloring agents may be added.

The pharmaceutical compositions of this invention may also be administered in the form of suppositories for rectal administration. These compositions can be prepared by mixing a compound of this invention with a suitable non-irritating excipient which is solid at room temperature but liquid at the rectal temperature and therefore will melt is the rectum to release the active components. Such materials include, but are not limited to, cocoa butter, beeswax and polyethylene glycols.

Topical administration of the pharmaceutical compositions of this invention is especially useful when the desired treatment involves areas or organs readily accessible by topical application. For application topically to the skin, the pharmaceutical composition should be formulated with a suitable ointment containing the active components suspended or dissolved in a carrier. Carriers for topical administration of the compounds of this invention include, but are not limited to, mineral oil, liquid petroleum, white petroleum, propylene glycol, polyoxyethylene polyoxypropylene compound, emulsifying wax and water. Alternatively, the pharmaceutical composition can be formulated with a suitable lotion or cream containing the active compound suspended or dissolved in a carrier. Suitable carriers include, but are not limited to, mineral oil, sorbitan monostearate, polysorbate 60, cetyl esters wax, cetearyl alcohol, 2-octyldodecanol, benzyl alcohol and water. The pharmaceutical compositions of this invention may also be topically applied to the lower intestinal tract by rectal suppository formulation or in a suitable enema formulation. Topically-transdermal patches are also included in this invention.

The pharmaceutical compositions of this invention may be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

Dosage levels of between about 0.01 and about 100 mg/kg body weight per day, preferably between about 0.5 and about 50 mg/kg body weight per day of the active ingredient compound are useful in the prevention and treatment of viral infection, including HIV infection. Typically, the pharmaceutical compositions of this invention will be administered from about 1 to about 5 times per day or alternatively, as a continuous infusion. Such administration can be used as a chronic or acute therapy. The amount of active ingredient that may be combined with the carrier materials to produce a single dosage form will vary depending upon the host treated and the particular mode of administration. A typical preparation will contain from about 5% to about 95% active compound (w/w). Preferably, such preparations contain from about 20% to about 80% active compound.

Upon improvement of a patient's condition, a maintenance dose of a compound, composition or combination of this invention may be administered, if necessary. Subsequently, the dosage or frequency of administration, or both, may be reduced, as a function of the symptoms, to a level at which the improved condition is retained when the symptoms have been alleviated to the desired level, treatment should cease. Patients may, however, require intermittent treatment on a long-term basis upon any recurrence of disease symptoms.

As the skilled artisan will appreciate, lower or higher doses than those recited above may be required. Specific dosage and treatment regimens for any particular patient will depend upon a variety of factors, including the activity of the specific compound employed, the age, body weight, general health status, sex, diet, time of administration, rate of excretion, drug combination, the severity and course of the infection, the patient's disposition to the infection and the judgment of the treating physician.

The compounds of this invention are also useful as commercial reagents which effectively bind to aspartyl proteases, particularly HIV aspartyl protease. As commercial reagents, the compounds of this invention, and their derivatives, may be used to block proteolysis of a target peptide or may be derivatized to bind to a stable resin as a tethered substrate for affinity chromatography applications. These and other users which characterize commercial aspartyl protease inhibitors will be evident to those of ordinary skill in the art.

As used herein, the compounds according to the invention are defined to include pharmaceutically acceptable derivatives or prodrugs thereof. A "pharmaceutically acceptable derivative" or "pharmaceutically acceptable prodrug" means any pharmaceutically acceptable salt, ester, salt of an ester, or other derivative of a compound of this invention which, upon administration to a recipient, is capable of providing (directly or indirectly) a compound of this invention or an active metabolite or residue thereof. Particularly favored derivatives and prodrugs are those that increase the bioavailability of the compounds of this invention when such compounds are administered to a mammal (e.g., by allowing an orally administered compound to be more readily absorbed into the blood) or which enhance delivery of the parent compound to a biological compartment (e.g., the brain or lymphatic system) relative to the parent species.

The compounds according to the invention may be used in the form of salts derived from inorganic or organic acids. Included among such acid salts, for example, are the following: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, flucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectianate, persulfate, phenylproprionate, picrate, pivalate, propionate, succinate, tartrate, thiocyanate, tosylate and undecanoate. Other acids, such as oxalic, while not in themselves pharmaceutically acceptable, may be employed in the preparation of salts useful as intermediates in obtaining the compounds of the invention and their pharmaceutically acceptable acid addition salts.

Salts derived from appropriate bases include alkali metal (e.g. sodium), alkaline earth metal (e.g., magnesium), ammonium and ⁺NW4 (wherein W is C₁₋₄ alkyl). Physiologically acceptable salts of a hydrogen atom or an amino group include salts or organic carboxylic acids such as acetic, lactic, tartaric, malic, isethionic, lactobionic and succinic acids; organic sulfonic acids such as methanesulfonic, ethanesulfonic, benzenesulfonic and p-toluenesulfonic acids and inorganic acids such as hydrochloric, sulfuric, phosphoric and sulfamic acids. Physiologically acceptable salts of a compound with a hydroxy group include the anion of said compound in combination with a suitable cation such as Na⁺, NH₄⁺, and NW₄⁺ (wherein W is a C₁₋₄ alkyl group).

Pharmaceutically acceptable salts include salts of organic carboxylic acids such as ascorbic, acetic, citric, lactic, tartaric, malic, maleic, isothionic, lactobionic, p-aminobenzoic and succinic acids; organic sulphonic acids such as methanesulphonic, ethanesulphonic, benzenesulphonic and p-toluenesulphonic acids and inorganic acids such as hydrochloric, sulphuric, phosphoric, sulphamic and pyrophosphoric acids.

For therapeutic use, salts of the compounds according to the invention will be pharmaceutically acceptable. However, salts of acids and bases which are non-pharmaceutically acceptable may also find use, for example, in the preparation or purification of a pharmaceutically acceptable compound.

Preferred salts include salts formed from hydrochloric, sulfuric, acetic, succinic, citric and ascorbic acids.

Preferred esters of the compounds according to the invention are independently selected from the following groups: (1) carboxylic acid esters obtained by esterification of the hydroxy groups, in which the non-carbonyl moiety of the carboxylic acid portion of the ester grouping is selected from straight or branched chain alkyl (for example, acetyl, n-propyl, t-butyl, or n-butyl), alkoxyalkyl (for example, methoxymethyl), aralkyl (for example, benzyl), aryloxyalkyl (for example, phenoxymethyl), aryl (for example, phenyl optionally substituted by, for example, halogen, C₁₋₄alkyl, or C₁₋₄alkoxy or amino); (2) sulfonate esters, such as alkyl- or aralkylsulfonyl (for example, methanesulfonyl); (3) amino acid esters (for example, L-valyl or L-isoleucyl); (4) phosphonate esters and (5) mono-, di- or triphosphate esters. The phosphate esters may be further esterified by, for example, a C₁₋₂₀ alcohol or reactive derivative thereof, or by a 2,3-di (C₆₋₂₄) acyl glycerol.

In such esters, unless otherwise specified, any alkyl moiety present advantageously contains from 1 to 18 carbon atoms, particularly from 1 to 6 carbon atoms, more particularly from 1 to 4 carbon atoms, Any cycloalkyl moiety present in such esters advantageously contains from 3 to 6 carbon atoms. Any aryl moiety present in such esters advantageously comprises a phenyl group.

Any reference to any of the above compounds also includes a reference to a pharmaceutically acceptable salts thereof.

The compounds according to the invention are especially useful for the treatment of AIDS and related clinical conditions such as AIDS related complex (ARC), progressive generalized lymphadenopathy (PGL), Kaposi's sarcoma, thrombocytopenic purpura, AIDS-related neurological conditions such as AIDS dementia complex, multiple sclerosis or tropical paraperesis, and also anti-HIV antibody-positive and HIV-positive conditions, including such conditions in asymptomatic patients.

In a further aspect of the invention there are provided the compounds according to the invention for use in medical therapy particularly for the treatment or prophylaxis of viral infections such as HIV infections.

According to another aspect, the present disclosure provides a method for the treatment or prevention of the symptoms or effects of a viral infection in an infected animal, for example, a mammal including a human, which comprises treating said animal with a therapeutically effective amount of a compound according to the invention. According to a particular embodiment of this aspect of the invention, the viral infection is an HIV infection. A further aspect of the disclosure includes a method for the treatment or prevention of the symptoms or effects of an HBV infection.

The compounds according to the invention may also be used in adjuvant therapy in the treatment of HIV infections or HIV-associated symptoms or effects, for examples Kaposi's sarcoma.

The present disclosure further provides a method for the treatment of a clinical condition in an animal, for example, a mammal including a human which clinical condition includes those which have been discussed in the introduction hereinbefore, which comprises treating said animal with a therapeutically effective amount of a compound according to the invention. The present disclosure also includes a method for the treatment or prophylaxis of any of the aforementioned infections or conditions.

Reference herein to treatment extends to prophylaxis as well as the treatment of established infections or symptoms.

The above compounds according to the invention and their pharmaceutically acceptable derivatives may be employed in combination with other therapeutic agents for the treatment of the above infections or conditions. Combination therapies according to the present invention comprise the administration of at least one compound of the formula (I) or a pharmaceutically acceptable derivative thereof and at least one other pharmaceutically active ingredient. The active ingredient(s) and pharmaceutically active agents may be administered simultaneously in either the same or different pharmaceutical formulations or sequentially in any order. The amounts of the active ingredient(s) and pharmaceutically active agent(s) and the relative timings of administration will be selected in order to achieve the desired combined therapeutic effect. Preferably the combination therapy involves the administration of one compound according to the invention and one of the agents mentioned herein below.

Examples of such further therapeutic agents include agents that are effective for the treatment of viral infections or associated conditions such as (1 alpha, 2 beta, 3 alpha)-9-(2,3-bis(hydroxymethyl) cyclobutyl]guanine [(-)BHCG, SQ-34514], oxetanocin-G (3,4-bis-(hydroxymethyl)-2-oxetanosyl]guanine), acyclic nucleosides (e.g. acyclovir, yalaciclovir, famciclovir, ganciclovir, penciclovir), acyclic nucleoside phosphonates (e.g. (S)-1-(3-hydroxy-2-phosphonylmethoxypropyl)cytosine (HPMPC) and PMEA analogs thereof, ribonucleotide reductase inhibitors such as 2-acetylpyridine 5-[(2-chloroanilino)thiocarbonyl) thiocarbonohydrazone, 3'azido-3'-deoxythymidine, others 2',3'-dideoxynucleosides such as 2',3'-dideoxycytidine, 2',3'-dideoxyadenosine, 2',3'-dideoxyinosine, 2',3'-didehydrothymidine, protease inhibitors such as indinavir, ritonavir, nelfinavir, [3S-[3R*(1R*, 2S*)]]-[3[[(4-aminophenyl) sulfonyl] (2-methylpropyl) amino) -2-hydroxy-1-(phenylmethyl)propyl]-tetrahydro-3-furanyl ester (141W94), oxathiolane nucleoside analogues such as (-)-cis-1-(2-hydroxymethyl)-1,3-oxathiolane 5-yl)-cytosine (lamivudine) or cis-1-(2-(hydroxymethyl)-1,3-oxathiolan-5-yl)-5-fluorocytosine (FTC), 3'-deoxy-3'-fluorothymidine, 5-chloro-2',3'-dideoxy-3'-fluorouridine, (-)-cis-4-[2-amino-6-(cyclopropylamino)-9H-purin-9-yl]-2-cyclopentene-1-methanol, ribavirin, 9-[4-hydroxy-2-(hydroxymethyl)but-1-yl]-guanine (H2G), tat inhibitors such as 7-chloro-5-(2-pyrryl)-3H-1,4-benzodiazepin-2-(H) one (Ro5-3335), 7-chloro-1,3-dihydro-5-(1H-pyrrol-2yl)-3H-1,4-benzodiazepin-2-amine (Ro24-7429), interferons such as α-interferon, renal excretion inhibitors such as probenecid, nucleoside transport inhibitors such as dipyridamole; pentoxifylline, N-acetylcysteine (NAC), Procysteine, a -trichosanthin, phosphonoformic acid, as well as immunomodulators such as interleukin II or thymosin, granulocyte macrophage colony stimulating factors, erythropoetin, soluble CD₄ and genetically engineered derivatives thereof, or non-nucleoside reverse transcriptase inhibitors (NNRTIs) such as nevirapine (BI-RG-587), loviride (α -APA) and delavuridine (BHAP), and phosphonoformic acid, and 1,4-dihydro-2H-3,1-benzoxazin-2-ones NNRTIs such as (-)-6-chloro-4-cyclopropylethynyl-4-trifluoromethyl-1,4-dihydro-2H-3,1-benzoxazin-2-one (L-743,726 or DMP-266), and quinoxaline NNRTIs such as isopropyl (2S)-7-fluoro-3,4-dihydro-2-ethyl-3-oxo-1(2H)-quinoxalinecarboxylate (HBY1293).

More preferably the combination therapy involves the administration of one of the above mentioned agents and a compound within one of the preferred or particularly preferred sub-groups within formula (I) as described above. Most preferably the combination therapy involves the joint use of one of the above named agents together with one of the compounds of formula (I) specifically named herein.

The present invention further includes the use of a compound according to the invention in the manufacture of a medicament for simultaneous or sequential administration with at least one other therapeutic agent, such as those defined herein before.

In order that this invention may be more fully understood, the following examples are set forth. These examples are for the purpose of illustration only and are not to be construed as limiting the scope of the invention in any way. Compounds for which experimentals are not shown may be made through similar methodologies.

### Example 1

### Synthesis of BOC Benzyl Tyrosine Based

### Weinreb Amide (Scheme 1, Step a)

N-t-BOC-O-Benzyl-L-Tyrosine(1, Sigma) (25 g, 67.3 mmol) was combined with anhydrous DMF (200 ml) and cooled to 0°C under a N₂ atmosphere. HOBT (15.5 g, 114.4 mmol, 1.7 eq.) and EDC (15.5 g, 80.8 mmol, 1.2 eq.) were added as solids and stirred to dissolve. Diisopropylethylamine (17.6 ml, 101 mmol, 1.5 'eq.) and 4-dimethylaminopyridine (0.001 g) were added and the reaction was stirred for 50 minutes at 0°C. N,O-Dimethylhydroxylamine hydrochloride (8..5 g, 87.5 mmol, 1.3 eq.) was added as a solid and the reaction was stirred for 10 minutes at 0°C then allowed to warm to room temperature and stirred overnight. After 18 hours at room temperature, the reaction was cooled to 0°C, and quenched with 200 mL of a 5% sodium bicarbonate solution. The reaction was extracted twice with EtOAc. The combined organics were washed with five times with water, and then brine, dried over MgSO₄, filtered and the solvent was removed *in vacuo.* The yield was 28g of amide which was used as is.

HPLC (5-100% CH₃CN/water) showed 1 peak at 10.77 min and the NMR (CDCl₃) was consistent with the expected structure.

### Example 2

### BOC Benzyl Tyrosine derived Vinyl Ketone (Scheme 1, Step b)

N-t-BOC-O-Benzyl-L-Tyrosine Weinreb amide (18.8 g, 45.3 mmoles) was combined with anhydrous THF (200 ml) and cooled to -78°C. A vinyl lithium solution (2.3 M, 50 ml, 2.5 eq.) was added via addition funnel dropwise over 20 minutes at -78°C. Ten mL of anhydrous THF was added to rinse the funnel. The reaction was stirred at -78°C under a N₂ atmosphere. HPLC at 1.5 hours showed the reaction was ∼50% complete. Another 1.0 eq (20ml) of vinyl lithium was added over 10 minutes at -78°C and washed in with 15mL of THF. The reaction stirred overnight at -78°C. After 18 hours, HPLC showed ∼15% Weinreb amide left. Another 0.2 eq (4 ml) of vinyl lithium was added at -78°C. After 26 hours at -78°C, the reaction was quenched with a slow addition of 300mL of 1N HCl. The reaction was partitioned between EtOAc and water. The aqueous phase was extracted with EtOAc. The combined organics were washed consecutively with saturated bicarbonate solution and brine, dried over MgSO₄, filtered and the solvent was removed *in vacuo.* The yield was 19.9 g crude material.

The material was purified by flash chromatography (gradient: CH₂Cl₂ to 10%EtOAc/CH₂Cl₂) to give 13.5g (78%) of pure material.
HPLC (5-100% CH₃CN/water) showed 1 peak at 13.37 min and the LC/MS showed 1 peak with an M+H= 382.4 for the desired compound.

### Example 3

### BOC Benzyl Tyrosine derived Allyl Alcohol (Scheme 1, step c, compound 2)

N-t-BOC-O-benzyl-L-tyrosine vinyl ketone (13.5 g, 35.4 mmol) was combined with methanol (120 ml) and Methylene Chloride(30 mL) and cooled to 0°C. Cerium chloride heptahydrate (14.5 g, 39 mmol, 1.1 eq.) was then added as a solid. The reaction was stirred at 0°C for 5 minutes and then cooled to -78°C. A solution of sodium borohydride (2.0 g, 53.1 mmol, 1.5 eq) in 40 mL of MeOH was cooled to -78°C and canulated into the reaction dropwise over 40 minutes. The reaction became a thick white suspension and 50mL of MeOH was added to aid stirring. The reaction was stirred at -78°C for 1.5 hours and was then quenched at -78°C with 150 mL of a saturated ammonium chloride solution. The reaction was extracted three times with EtOAc, and the combined organics were washed with saturated bicarbonate solution, followed by brine, dried over Na₂SO₄, filtered and the solvent was removed in vacuo to give 13.6 g crude material.

Proton NMR (CDCl₃) shows a 7:3 ratio of diastereomers. The material was purified via chromatography (4:5:1, Hexanes:CH₂Cl₂:EtOAc) to give 5.4 g of.desired material (83:17 ratio of diastereomers) as well as 7.3 g of allyl alcohol as a mix of diastereomers to be repurified. Proton NMR (CDCl₃) was consistent with structure for the desired material.

### Example 4

### BOC Benzyl Tyrosine Allyl Alcohol

### (THP protected) Scheme 1, step e:

BOC benzyl tyrosine allyl alcohol (1.59 g, 4.1 mmole) was dissolved in 10 mL of anhydrous CH₂Cl₂. Dihydropyran (500 µL, 5.4 mmol, 1.3eq.) and pyridinium paratoluenesulfonate (210 mg, 0.8 mmol, 0.2 eq.) was added and the reaction was stirred at room temperature under a N₂ atmosphere. After 19 hours, the solvent was removed in vacuo and the residue was partitioned between EtOAc and a 10% citric acid solution. The organics were separated, washed with brine, and then saturated bicarbonate solution, dried over Na₂SO₄, filtered and the solvent was removed *in vacuo* to give 1.98 g crude material as a white solid.

The material was purified via chromatography (25% EtOAc/Hexanes, (with 0.5ml NEt₃/L)) to give 1.85 g (96%) of desired material. NMR (CDCl₃) was consistent with structure as a mix of diastereomers (1:1) at THP alcohol.

### Example 5

### BOC Benzyl Tyrosine Diol (THP protected)

### (Scheme 1, steps f and g, compound 3)

THP protected BOC benzyl tyrosine allyl alcohol (1.5 g, 3.2 mmol) was dissolved in methanol (5 ml) and methylene chloride (20 mL) and cooled to -78°C. Ozone was bubbled into the stirred solution for 1.5 hours at -78°C. The solution was then flushed with nitrogen to remove the ozone. Sodium borohydride (920 mg, 25.6 mmol, 8 eq.) was then added in small portions over 5 minutes at -78°C. Methanol (35 mL) was added and the reactrion was stirred at 78°C four 5 minutes; then was slowly warmed to 0°C. Vigorous bubbling began at --20°C. After 1.5 hours at 0°C the reaction was quenched with saturated bicarbonate solution and extracted with CH₂Cl₂. The combined organics were washed with brine, dried over Na₂SO₄, filtered and the solvent was removed *in vacuo* to give 1.49 g crude material.

The material was purified via chromatography (EtOAc/Hexanes, 20%-30%-40%, containing 0.5ml NEt₃/L) to give 1.15 g (76%) of desired material. HPLC (5-100% CH₃CN/water) showed 1 peak at 13.81 min and the proton NMR (CDCl₃) was consistent with structure as a mix of diastereomers (-1:1) at the THP alcohol.

### Example 6

### Epoxide (4)

THP-protected diol (3) (0.40 g, 0.85 mmol) was combined with a catalytic amount of *p*-toluenesulfonic acid (0.004 g) in methanol (20 mL) under a N₂ atmosphere. After stirring at room temperature for *ca*. 15 minutes, the initial white suspension dissolved completely. Stirring at room temperature was continued for *ca*. 1 hour, after which time complete disappearance of starting material (3) was confirmed by TLC. The solvent was removed *in vacuo* to give diol as a white solid, combined with residual *p*-toluenesulfonic acid. The crude diol was dissolved in anhydrous dichloromethane (15 mL), and trimethylorthoacetate (0.130 mL, 1.02 mmol) was added dropwise with stirring. Upon addition of trimethylorthoacetate, the cloudy solution became colourless. After stirring at room temperature for *ca*. 1 hour, TLC again indicated complete disappearance of starting material. The solvent was removed *in vacuo* to give the desired cyclic orthoacetate as a viscous white oil, which was re-dissolved in anhydrous DCM (15 mL). Trimethysilyl chloride (0.129 mL, 1.02 mmol) was added dropwise with stirring. After 1.5 hours at room temperature generally no further starting material remained. The solvent was removed *in vacuo* to give the desired chloroacetates as a yellow oil, which was dissolved in methanol (20 mL). Cesium carbonate (0.48 g, 1.48 mmol) was added in one portion, and the solution was stirred at room temperature for 2 hours, after which time, TLC confirmed the absence of any starting material. The solvent was removed *in vacuo* to give a pale yellow oil, which was partitioned between saturated aqueous ammonium chloride solution (30 mL) and DCM (30 mL). The organic layer was taken, and the aqueous layer reextracted with DCM (2 x 30 mL). The combined organic extracts were dried over MgSO₄, and the solvent removed *in vacuo* to give crude epoxide (4) as a yellow oil. This material was either used in the crude form, or could be purified by flash column chromatography (3:7 ethyl acetate/hexane to 4:1 ethyl acetate/methanol) to give epoxide (4) as a white solid: R_{f} = 0.60 (3:7 ethyl acetate/hexane); ¹H NMR (CDCl₃) 7.49-7.29 (5H, in), 7.14 (2H, d, J = 8.3 Hz), 6.93 (2H, d, J = 8.3 Hz), 5.05 (2H, s), 4.44 (1H, br. s), 3.64 (1H, br. s), 2.97-2.86 (2H, m), 2.85-2.72 (3H, m), 1.39 (9H, s); coupled LCMS showed the product as a single major peak with *m*/*z* 370 [M+H]⁺ or *m*/*z* 312 [M+H-^{t}Bu]⁺ at R_{T} 2.84 min; HPLC (205 nm) over an extended 20 minute run time showed the crude material to be a 9:1 mixture of diastereoisomeric epoxides at R_{T}s of 14.2 min. (*major diastereoisomer*) & 14.3 min. (*minor diastereoisomer*).

### Example 7

### Isobutylamino Alcohol (Scheme 1, step j)

Crude epoxide (4) (0.37 g, 0.85 mmol) was combined with isobutylamine *(large excess,* 4 mL) in ethanol (4 mL) under a N₂ atmosphere. The reaction mixture was heated to reflux with stirring for 2.5 hours. The solvent was removed in vacuo to give a pale yellow oily residue. Trituration with hexane gave the isobutylamino alcohol (0.285 g, 75%) as a white solid: R_{f} = 0.05 (3:7 ethyl acetate/hexane); ¹H NMR (CDCl₃) 7.47-7.29 (5H, m), 7.15 (2H, d, J = 8.5 Hz) , 6.91 (2H, d, J = 8.5 Hz) , 5.04 (2H, s), 4.69 (1H, br. d; J = 8.8 Hz), 3.76 (1H, br. s), 3.49-3.40 (1H, m), 2.91 (1H, dd, J = 14. 1, 4.7 Hz), 2.87-2.77 (1H, m), 2.67 (2H, d, J = 4.7 Hz), 2.40 (2H, d, J = 6.7 Hz), 1.71 (1H, septet, J = 6.7 Hz), 1.36 (9H, s), 0.91 (3H, d, J = 6.7 Hz), 0.90 (3H, d, J = 6.7 Hz), O*H* and N*H* signals not observed; distinct *minor diastereoisomer* signals observed: 4.55 (1H, d, J = 8.7 Hz), 2.49 (2H, d, J = 6.6 Hz); NMR integration showed the triturated product to be a 9:1 mixture of diastereoisomeric isobutylamino alcohols; coupled LCMS showed the product as a single major peak with *m*/*z* 443 [M+H]⁺ at R_{T} 2.41 min.

### Example 8

### Boc methylenedioxybenzenesulfonamide benzyl ether (Scheme 1, Compound 5e)

Isobutylamino alcohol (0.170 g, 0.38 mmol) was combined with methylenedioxybenzenesulfonyl chloride (0.085 g, 0.38 mmol) in anhydrous DCM (5 mL) under a N₂ atmosphere. The solution was cooled to 0°C using an ice bath, and diisopropylethylamine (0.20 mL, 1.20 mmol) was added dropwise, and the reaction was allowed to warm to room temperature with stirring for 4 hours. The solvent was removed in vacuo, and the resultant pale yellow oil was purified by flash column chromatography (3:7 ethyl acetate/hexane) to give Boc methylenedioxybenzenesulfonamide benzyl ether (0.185 g, 75%) as a white foam: R_{f} = 0.30 (3:7 ethyl acetate/hexane); ¹H NMR (CDCl₃) 7.47-7.29 (6H, m), 7.21-7.11 (3H, m), 6.92 (2H, d, J = 8.4 Hz), 6.88 (1H, d, J = 8.2 Hz), 6.07 (2H, s), 5.04 (2H, s), 4.67-4.58 (1H, m), 3.97-3.85 (1H, m), 3.82-3.56 (2H, m), 3.10-3.02 (2H, m), 2.98-2.72 (4H, m), 1.84 (1H, septet, J = 6.5 Hz), 1.36 (9H, s), 0.91 (3H, d, J = 6.5 Hz), 0.87 (3H, d, J = 6.5 Hz); coupled LCMS showed the product as a single major peak with *m*/*z* 627 [M+H]⁺ at R_{T} 3.16 min.

### Example 9

### Boc m-nitrobenzenesulfonamide

### Benzyl Ether (Scheme 1, Compound 5c)

The isobutylamino alcohol (0.059 g, 0.13 mmol), as made in Scheme I by the addition of i-RuNH₂ to compound 4, was combined with m-nitrobenzenesulfonyl chloride (0.044 g, 0.20 mmol) in anhydrous DCM (2 mL) under a N₃ atmosphere. Diisopropylethylamine (0.070 mL, 0.40 mmol) was added dropwise, and the reaction was stirred at room temperature for 48 hours. The solvent was removed in vacuo, and the resultant yellow oil was purified by flash column chromatography (3:7 ethyl acetate/hexane) to give Boc m-nitrobenzenesulfonamide benzyl (5) (0.050 g, 61%) as a colourless oil: R_{f} = 0.31 (3:7 ethyl acetate/hexane); ¹H NMR (CDCl₃) 8.63 (1H, d, J = 1.8 Hz), 8.41 (1H, d, J = 8.1 Hz), 8.10 (1H, d, J = 6.8 Hz), 7.72 (1H, t, J = 7.9 Hz), 7.50-7.28 (5H, m), 7.15 (2H, d, J = 8.6 Hz), 6.92 (2H, d, J = 8.6 Hz), 5.00 (2H, s), 4.65-4.55 (1H, m), 3.99-3.84 (1H, m), 3.83-3.74 (1H, m), 3.74-3.64 (1H, m), 3.21 (2H, d, J = 5.4 Hz), 3.01 (2H, d, J = 7.2 Hz), 2.93-2.80 (2H, m), 1.88 (1H, septet, J = 6.4 Hz), 1.36 (9H, s), 0.91-0.75 (6H, m).

### Example 10

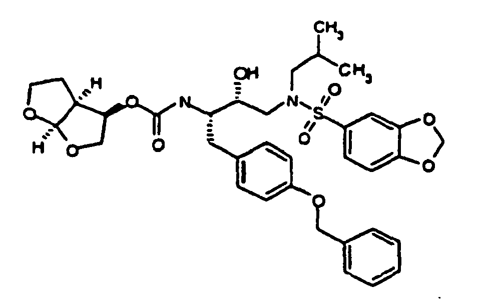

### Bis-THF methylenedioxybenzenesulfonamide benzyl ether (46)

Boc methylenedioxybenzenesulfonamide benzyl ether (0.040 g, 0.064 mmol) was dissolved in DCM (2 mL). Trifluoroacetic acid (1 mL) was added dropwise, and the reaction was stirred at room temperature for 1 hour. The solvent was removed *in vacuo,* and the resultant orange oil was dissolved in DCM (1.5 mL) and the solution cooled to 0 °C using an ice bath. Diisopropylethylamine (0.33 mL, 1.92 mmol) was added dropwise with stirring, followed by (3R, 3aS, 6aR) hexahydrofuro[2,3-*b*]furan-2-yl 4-nitrophenyl carbonate (0.021 g, 0.071 mmol) in one portion as a solid. After 5 minutes, the ice bath was removed and the reaction mixture was allowed to warm to room temperature with stirring overnight. The solvent was removed *in vacuo,* and the resultant yellow oil was purified by flash column chromatography (1:1 ethyl acetate/hexane) to give *bis*-THF methylenedioxybenzenesulfonamide benzyl ether (46) (0.035 g, 80%) as a white foam: R_{f} = 0.31 (1:1 ethyl acetate/hexane) ; ¹H NMR (CDCl₃) 7.45-7.29 (6H, m), 7.17 (1H, d, J = 1.8 Hz), 7.13 (2H, d, J = 8.3 Hz), 6.90 (2H, d, J = 8.3 Hz), 6.94-6.86 (1H, m), 6.07 (2H, s), 5.66 (1H, d, J = 5.2 Hz), 5.10-4.98 (1H, m), 5.02 (2H, s), 4.94 (1H, d, J = 8.5 Hz), 3.96 (1H, dd, J = 9.6, 6.4 Hz), 3.89-3.78 (3H, m), 3.76-3.65 (2H, m), 3.18-3.09 (1H, m), 3.04-2.85 (4H, m), 2.82-2.70 (2H, m), 1.90-1.77 (1H, m), 1.73-1.48 (2H, m), 0.93 (3H, d, J = 6.5 Hz), 0.89 (3H, d, J = 6.5 Hz), O*H* signal not observed; coupled LCMS showed the product as a single major peak with *m*/*z* 683 [M+H]⁺; HPLC (205 nm) showed the material as a single major peak at R_{T} of 2.73 min. (purity = 96%).

### Example 11

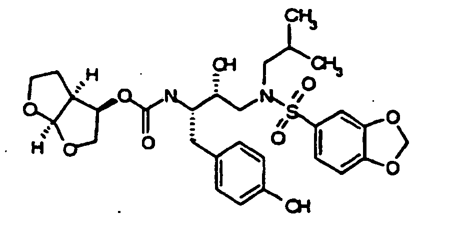

### Bis-THF methylenedioxybenzenesulfonamide free phenol (47)

A solution of *bis*-THF methylenedioxybenzenesulfonamide benzyl ether (46) (0.022 g, 0.032 mmol) and 10% palladium on carbon (wet; Degussa variant) (0.008 g) in degassed ethyl acetate (10 mL) was stirred under a balloon of hydrogen. The reaction was monitored by TLC, and after 20 hours the starting material (47) had not been consumed. A fresh portion of 10% palladium on carbon (wet; Degussa variant) (0.008 g) was added to the reaction mixture, and the reaction stirred under a balloon of hydrogen for a further 4.5 hours. The reaction mixture was filtered through a pad of celite, and the filtrate was dried *in vacuo* to give a pale yellow oil. Flash column chromatography (1:1 ethyl acetate/hexane) gave *bis*-THF methylenedioxybenzenesulfonamide free phenol (47) (0.008 g, 42%) as a colourless oil: R_{f} = 0.44 (1:1 ethyl acetate/hexane); ¹H NMR (CDCl₃); 7.33 (1H, dd, J = 8.2, 1.7 Hz), 7.17 (1H, s), 7.07 (2H, d, J = 8.1 Hz), 6.90 (1H, d, J = 8.2 Hz), 6.74 (2H, d, J = 8.1 Hz), 6.09 (2H, s), 5.66 (1H, d, J = 5.2 Hz), 5.10-5.00 (2H, m), 4.05-3.68 (7H, m), 3.18-3.07 (1H, m), 3.06-2.90 (4H, m), 2.87-2.68 (2H, m), 1.89-1.48 (3H, m), 0.93 (3H, d, J = 6.6 Hz), 0.89 (3H, d, J = 6.6 Hz); *OH* signal not observed; coupled LCMS showed the product as a single major peak with *m*/*z* 593 [M+H]⁺; HPLC (205 nm) showed the material as a single major peak at R_{T} of 1.94 min. (purity = 98%).

### Example 12

### Boc methylenedioxybenzenesulfonamide free phenol (Scheme 1, Compound 6e)

A solution of Boc methylenedioxybenzenesulfonamide benzyl ether (0.063 g, 0.101 mmol) and 10% palladium on carbon (wet; Degussa variant) (0.024 g) in degassed ethyl acetate (10 mL) was stirred under a balloon of hydrogen. The reaction was monitored by TLC, and after 2 hours the starting material had not been consumed. A fresh portion of 10% palladium on carbon (wet; Degussa variant) (0.024 g) was added to the reaction mixture, and the reaction stirred under a balloon of hydrogen for a further 5 hours. The reaction mixture was filtered through a pad of celite, and the filtrate was dried *in vacuo* to give a colourless oil. Flash column chromatography (1:1 ethyl acetate/hexane) gave Boc methylenedioxybenzenesulfonamide free phenol (6) (0.036 g, 60%) as a white foam: R_{f} = 0.74 (1:1 ethyl acetate/hexane); ¹H NMR (CDCl₃) ; 7.32 (1H, d, J = 8.3 Hz), 7.17 (1H, s), 7.12-7.02 (2H, m), 6.88 (1H, d, J = 8.3 Hz), 6.78-6.69 (2H, m), 6.08 (2H, s), 4.75 (1H, d, J = 8.1 Hz), 3.88-3.62 (3H, m), 3.49 (1H, s), 3.09-3.01 (2H, m), 2.97-2.85 (2H, m), 2.84-2.72 (2H, m), 1.84 (1H, septet, J = 6.6 Hz), 1.37 (9H, s), 0.90 (3H, d, J = 6.6 Hz), 0.86 (3H, d, J = 6.6 Hz); coupled LCMS showed the product as a single major peak with *m*/*z* 537 [M+H]⁺ at R_{T} 2.50 min.

### Example 13

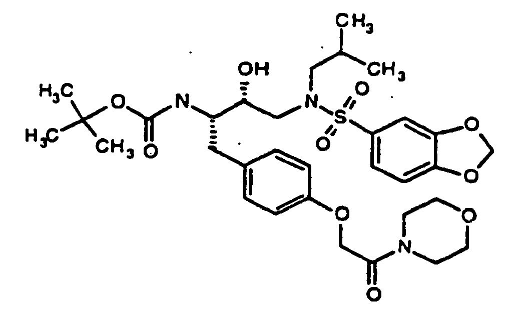

### Boc methylenedioxybenzenesulfonamide acetylmorpholine tethered product (21)

A solution of Boc methylenedioxybenzenesulfonamide free phenol (0.036 g, 0.067 mmol) in 1,4-dioxane (1 mL) was combined with cesium carbonate (0.055 g, 0.168 mmol) and 4-(2-chloroacetyl)morpholine (0.016 g, 0.100 mmol). The solution was heated to 85 °C with stirring for 2 hours, and was cooled and dried *in vacuo* to give a pale yellow oil. Flash column chromatography (ethyl acetate) gave Boc methylenedioxybenzenesulfonamide acetylmorpholine tethered product (21) (0.032 g, 71%) as a white foam: R_{f} = 0.51 (ethyl acetate); ¹H NMR (cDCl₃); 7.34 (1H, dd, J = 8.0, 1.7 Hz), 7.22-7.14 (3H, m), 6.93-6.84 (3H, m), 6.09 (2H, s), 4.70-4.59 (1H, m), 4.67 (2H, s), 3.97-3.90 (1H, m), 3.82-3.58 (10H, m), 3.12-3.04 (2H, m), 2.99-2.78 (4H, m), 1.84 (1H, septet, J = 6.6 Hz), 1.36 (9H, s), 0.91 (3H, d, J = 6.6 Hz), 0.88 (3H, d, J = 6.6 Hz); coupled LCMS showed the product as a single major peak with *m*/*z* 664 [M+H]⁺; HPLC (205 nm) showed the material as a single major peak at R_{T} of 2.38 min. (purity = 99%).

### Example 14

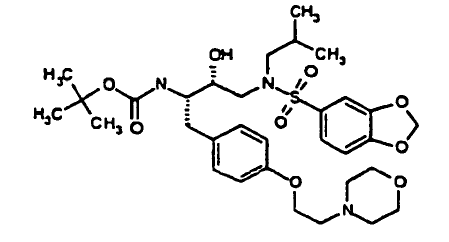

### Boc methylenedioxybenzenesulfonamide ethylmorpholine tethered product (17)

A solution of Boc methylenedioxybenzenesulfonamide free phenol (0.034 g, 0.063 mmol) in 1,4-dioxane (1 mL) was combined with cesium carbonate (0.052 g, 0.158 mmol) and *N*-(2-chloroethyl)morpholine (0.014 g, 0.095 mmol). The solution was heated to 85 °C with stirring for 5 hours, and was cooled and dried *in vacuo* to give a pale yellow oil. Flash column chromatography (4:1 ethyl acetate/hexane) gave Boc methylenedioxybenzenesulfonamide ethylmorpholine tethered product (17) (0.012 g, 29%) as a pale yellow oil: R_{f} = 0.32 (4:1 ethyl acetate/hexane); ¹H NMR (CDCl₃) ; 7.32 (1H, dd, J = 8.2, 1.5 Hz), 7.18 (1H, d, J = 1.5 Hz), 7.15 (2H, d, J = 8.3 Hz), 6.88 (1H, d, J = 8.2 Hz), 6.84 (2H, d, J = 8.3 Hz), 6.09 (2H, s), 4.63 (1H, d, J = 8.2 Hz), 3.93 (1H, br. s), 3.83-3.64 (7H, m), 3.06 (2H, d, J = 4.9 Hz), 2.98-2.76 (6H, m), 2.68-2.55 (4H, m), 2.54-2.48 (1H, m), 1.84 (1H, septet, J = 6.7 Hz), 1.35 (9H, s), 0.90 (3H, d, J = 6.7 Hz), 0.87 (3H, d, J = 6.7 Hz); coupled LCMS showed the product as a single major peak with *m*/*z* 650 [M+H]⁺; HPLC (205 nm) showed the material as a single major peak at R_{T} of 2.06 min. (purity = 92%).

### Example 15

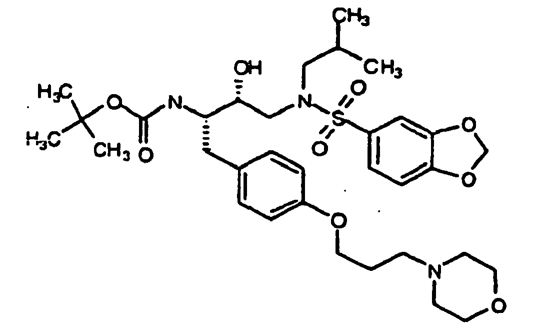

### Boc methylenedioxybenzenesulfonamide propylmorpholine tethered product (20)

A solution of Boc methylenedioxybenzenesulfonamide free phenol (0.038 g, 0.071 mmol) in 1,4-dioxane (1 mL) was combined with cesium carbonate (0.058 g, 0.177 mmol) and *N*-(3-chloropropyl)morpholine (0.017 g, 0.107 mmol). The solution was heated to 85 °C with stirring for 8 hours, and was cooled and dried *in vacuo* to give a pale yellow oil. Flash column chromatography (4:1 ethyl acetate/hexane) gave Boc methylenedioxybenzenesulfonamide propylmorpholine tethered product (20) (0.006 g, 13%) as a pale yellow oil: R_{f} = 0.15 (4:1 ethyl acetate/hexane); ¹H NMR (CDCl₃); 7.32 (1H, dd, J = 8.1, 1.8 Hz), 7.19 (1H, d, J = 1.8 Hz), 7.14 (2H, d, J = 8.4 Hz), 6.88 (1H, d, J = 8.1 Hz), 6.83 (2H, d, J = 8.4 Hz), 6.08 (2H, s), 4.62 (1H, br. s), 4.00 (2H, t, J = 6.3 Hz), 3.84-3.65 (8H, m), 3.61 (2H, t, J = 6.6 Hz), 3.10-3.03 (2H, m), 3.00-2.77 (3H, m), 2.64-2.35 (4H, m), 2.05-1.91 (2H, m), 1.85 (1H, septet, J = 6.6 Hz), 1.37 (9H, s), 0.90 (3H, d, J = 6.6 Hz), 0.87 (3H, d, J = 6.6 Hz); coupled LCMS showed the product as a single major peak with *m*/*z* 664 [M+H]⁺; HPLC (205 nm) showed the material as a single major peak at R_{T} of 2.23 min. (purity = 80%).

### Example 16

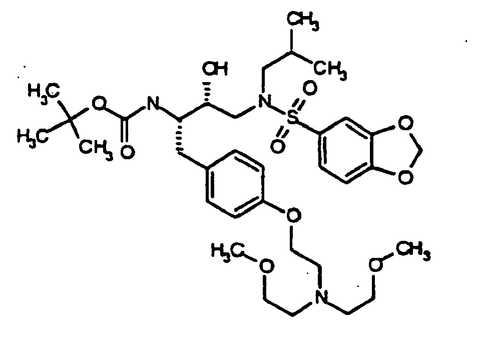

### Boc methylenedioxybenzenesulfonamide bis-methoxyethylamine tethered product (18)

A solution of Boc methylenedioxybenzenesulfonamide free phenol (0.034 g, 0.063 mmol) in 1,4-dioxane (1 mL) was combined with cesium carbonate (0.052 g, 0.158 mmol) and freshly prepared 2-[*N,N-bis*-(2-methoxyethyl)amino]ethyl chloride (ca. 0.031 g, ca. 0.016 mmol)⁻. The solution was heated to 85 °C with stirring for 3.5 hours, and was cooled and dried *in vacuo* to give a pale yellow oil. Flash column chromatography (ethyl acetate) gave Boc methylenedioxybenzenesulfonamide *bis*-methoxyethylamine tethered product (18) (0.024 g, 54%) as a white foam: R_{f} = 0.35 (ethyl acetate); ¹H NMR (CDCl₃) 7.32 (1H, dd, J = 8.2, 1.6 Hz), 7.18 (1H, d, J = 1.6 Hz), 7.14 (2H, d, J = 8.5 Hz), 6.88 (1H, d, J = 8.2 Hz), 6.83 (2H, d, J = 8.5 Hz), 6.08 (2H, s), 4.63 (1H, d, J = 7.7 Hz), 4.03 (2H, t, J = 6.1 Hz), 3.92 (1H, br. s), 3.81-3.73 (1H, m), 3.73-3.63 (1H, m), 3.50 (4H, t, J = 5.8 Hz), 3.34 (6H, s), 3.10-3.03 (2H, m), 3.01 (2H, t, J = 6.1 Hz), 2.98-2.75 (4H, m), 2.84 (4H, t, J = 5.8 Hz), 1.83 (1H, septet, J = 6.6 Hz), 1.35 (9H, s), 0.90 (3H, d, J = 6.6 Hz), 0.87 (3H, d, J = 6.6 Hz); coupled LCMS showed the product as a single major peak with *m*/*z* 696 [M+H]⁺; HPLC (205 nm) showed the material as a single major peak at R_{T} of 2.20 min. (purity = 100%).

### Example 17

### Boc methylenedioxybenzenesulfonamide 3-picolyl tethered product (intermediate en route to compound 53)

A solution of Boc methylenedioxybenzenesulfonamide free phenol (0.010 g, 0.020 mmol) in 1,4-dioxane (1 mL) was combined with cesium carbonate (0.015 g, 0.047 mmol), 3-(chloromethyl)pyridine (ca. 0.004 g, ca. 0.030 mmol; prepared by dissolving 10 mg of 3-(chloromethyl)pyridine hydrochloride in sodium hydroxide (1.5 mL) and diethyl ether (1.5 mL), the organic extract was dried over MgSO₄ and the solvent removed *in vacuo*) and potassium iodide (-1 mg, 0.006 mmol). The solution was heated to 60 °C with stirring for 8 hours, and was cooled and dried *in vacuo* to give a pale yellow oil. Flash column chromatography (3:7 ethyl acetate/hexane) gave Boc methylenedioxybenzenesulfonamide 3-picolyl tethered product (0.004 g, 34%) as a colourless oil: R_{f} = 0.05 (1:1 ethyl acetate/hexane); coupled LCMS showed the product as a single major peak with *m*/*z* 628 [M+H]⁺ at R_{T} of 2.27 min.

### Example 18

### Boc methylenedioxybenzenesulfonamide 2-picolyl tethered product (intermediate en route to 52) not encompassed in the present invention

A solution of Boc methylenedioxybenzenesulfonamide free phenol (0.010 g, 0.020 mmol) in 1,4-dioxane (1 mL) was combined with cesium carbonate (0.015 g, 0.047 mmol), 2-(chloromethyl)pyridine (ca. 0.004 g, ca. 0.030 mmol; prepared by dissolving 10 mg of 2-(chloromethyl)pyridine hydrochloride in sodium hydroxide (1.5 mL) and diethyl ether (1.5 mL), the organic extract was dried over MgSO₄ and the solvent removed *in vacuo*) and potassium iodide (~1 mg, 0.006 mmol). The solution was heated to 60 °C with stirring for 8 hours, and was cooled and dried *in vacuo* to give a pale yellow oil. Flash column chromatography (3:7 ethyl acetate/hexane) gave Boc methylenedioxybenzenesulfonamide 2-picolyl tethered product (0.004 g, 34%) as a colourless oil: R_{f} = 0.3 (1:1 ethyl acetate/hexane); coupled LCMS showed the product with *m*/*z* 628 [M+H]⁺ at R_{T} of 2.35 min.

### Example 19

### Boc methylenedioxybenzeaesulfonamide 4-picolyl tethered product (intermediate en route to compound 54) not encompassed in the present invention

A solution of Boc methylenedioxybenzenesulfonamide free phenol (0.010 g, 0.020 mmol) in 1,4-dioxane (1 mL) was combined with cesium carbonate (0.015 g, 0.047 mmol), 4-(chloromethyl)pyridine (ca. 0.004 g, ca. 0.030 mmol; prepared by dissolving 10 mg of 4-(chloromethyl)pyridine hydrochloride in sodium hydroxide (1.5 mL) and diethyl ether (1.5 mL), the organic extract was dried over MgSO₄ and the solvent removed *in vacuo*) and potassium iodide (-1 mg, 0.006 mmol). The solution was heated to 60 °C with stirring for 16 hours, and was cooled and dried *in vacuo* to give a pale yellow oil. Flash column chromatography (3:7 ethyl acetate/hexane) gave Boc methylenedioxybenzenesulfonamide 4-picolyl tethered product (0.004 g, 34%) as a colourless oil: R_{f} = 0.10 (2:3 ethyl acetate/hexane); coupled LCMS showed the product with *m*/*z* 628 [M+H]⁺ at R_{T} of 2.24 min.

### Example 20

### Boc methylenedioxybenzenesulfonamide 3-methyl-5-methylisoxazole tethered product

### (intermediate en route to compound 55) not encompassed in the present invention

A solution of Boc methylenedioxybenzenesulfonamide free phenol (0.010 g, 0.020 mmol) in 1,4-dioxane (1 mL) was combined with cesium carbonate (0.015 g, 0.047 mmol), 3-chloromethyl-5-methyl isoxazole (0.004 g, 0.028 mmol) and potassium iodide (-1 mg, 0.006 mmol). The solution was heated to 60 °C with stirring for 16 hour, and was cooled and dried *in vacuo* to give a pale yellow oil. Flash column chromatography (3:7 ethyl acetate/hexane) gave Boc methylenedioxybenzenesulfonamide 3-methyl-5-methylisoxazole tethered product (0.005 g, 42%) as a colourless oil: R_{f} = 0.25 (3:7 ethyl acetate/hexane); ¹H NMR (CDCl₃) 7.34 (1H, dd, J = 8.4, 1.8 Hz), 7.21-7.13 (3H, m), 6.90 (3H, m), 6.11 (1H, s), 6.09 (2H, s), 5.09 (2H, s), 4.65 (1H, d, J = 8.2 Hz), 3.92 (1H, br. s), 3.82-3.75 (1H, m), 3.73-3.63 (1H, m), 3.09-3.03 (2H, m), 2.98-2.80 (4H, m), 2.43 (3H, s), 1.84 (1H, septet, J = 6.6 Hz), 1.36 (9H, s), 0.91 (3H, d, J = 6.6 Hz), 0.88 (3H, d, J = 6.6 Hz).

### Example 21A

### Boc methylenedioxybenzenesulfonamide 1-methyl-3,5-dimethylpyrazole tethered product

### (intermediate en route to compound 56)

**not encompassed in the present invention** A solution of Boc methylenedioxybenzenesulfonamide free phenol (0.010 g, 0.020 mmol) in DMF (1 mL) was combined with anhydrous cesium carbonate (0.015 g, 0.047 mmol). Freshly prepared 1-chloromethyl-3,5-dimethylpyrazole hydrochloride (0.006 g, 0.033 mmol) in DMF (0.5 ml) was added dropwise. The solution was heated to 60 °C with stirring for 15 minutes, and was cooled and dried *in vacuo* to give a green oil. Flash column chromatography (1:1 ethyl acetate/hexane) gave Boc methylenedioxybenzenesulfonamide 1-methyl-3,5-dimethylpyrazole tethered product (0.002 g, 17%) as a colourless oil: R_{f} = 0.25 (1:1 ethyl acetate/hexane); coupled LCMS showed the product as a single major peak with *m*/*z* 645 [M+H]⁺ at R_{T} of 1.68 min.

### Example 21B

### Boc methylenedioxybenzenesulfonamide ethylpyrazole tethered product (intermediate en route to compound 57) not encompassed in the present invention

A solution of Boc methylenedioxybenzenesulfonamide free phenol (0.010 g, 0.020 mmol) in acetone (1 mL) was combined with cesium carbonate (0.015 g, 0.047 mmol), 1-(2-chloroethyl)pyrazole (0.004 g, 0.031 mmol) and sodium iodide (-1 mg, 0.007 mmol). The solution was heated to 55 °C with stirring for 60 hours, and was cooled and dried *in vacuo* to give a yellow oil. Flash column chromatography (1:3 ethyl acetate/hexane) gave Boc methylenedioxybenzenesulfonamide ethylpyrazole tethered product (0.0015 g, 13%) as a colourless oil: R_{f} = 0.20 (1:1 ethyl acetate/hexane); coupled LCMS showed the product as a single major peak with *m*/*z* 631 [M+H]⁺at R_{T} of 1.65 min.

### Example 27

### Boc Benzothiazole Sulfonamide Benzyl

### Ether (Compound 5g, Scheme 1)

The isobutylamino alcohol (0.50 g, 1.13 mmol) was combined with 2-aminobenzothiazole-6- sulfonyl chloride (0.373 g, 1.50 mmol) in anhydrous DCM (10 mL) under a N₂ atmosphere. Diisopropylethylamine (0.59 mL, 3.39 mmol) was added dropwise, and the reaction was stirred at room temperature for 42 hours. The solvent was removed *in vacuo*, and the resultant yellow oil was purified by flash column chromatography (4:1 ethyl acetate/hexane) to give Boc benzothiazole sulfonamide benzyl ether (5: D'= i-Bu, E = 2-aminobenzothiazole) (0.30 g, 42%) as a white solid: R_{f} = 0.60 (4:1 ethyl acetate/hexane); ¹H NMR (CDCl₃) 8.05 (1H, d, J = 1.8 Hz), 7.72 (1H, dd, J = 8.6, 1.8 Hz), 7.60 (1H, d, J = 8.6 Hz), 7.49-7.32 (5H, m), 7.19 (2H, d, J = 8.6 Hz), 6.94 (2H, d, J = 8.6 Hz), 5.67 (2H, br.s, NH₂), 5.07 (2H, s), 4.70 (1H, br.d, J = 7.7 Hz), 4.00 (1H, br.s, OH), 3.89-3.80 (1H, m), 3.80-3.66 (1H, m), 3.23-3.06 (2H, m), 3.06-2.77 (4H, m), 1.89 (1H, septet, J = 7.2 Hz), 1.38 (9H, s), 0.94 (3H, d, J = 6.3 Hz), 0.90 (3H, d, J = 6.3 Hz).

### Example 328

### Step 1:

### 3,3-Diethoxypropan-1-yl-4'-nitrophenyl carbonate

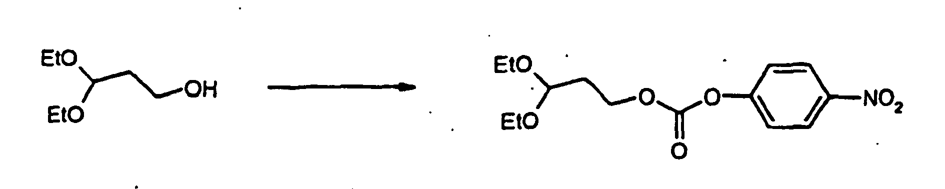

To an ice cold solution of 3,3-diethoxy-1-propanol (1.5 g, 10.1 mmol) and pyridine (1.0 mL, 12.2 mmol) in dichloromethane (30 mL) was added 4-nitrophenylchloroformate (2.24 g, 11.1 mmol). The reaction was allowed to warm to ambient temperature. After stirring for 18 h, the reaction was concentrated *in vacuo*, taken up in ethyl acetate (60 mL), washed with 5% aqueous citric acid (2x40 mL) followed by saturated sodium carbonate (3x40 mL), dried (magnesium sulfate) and concentrated. The residue was purified by silica gel chromatography (20% ethyl acetate in Hexane) to afford the title compound (1.05g, 33%) a an oil. ¹H NMR (CDCl₃) : δ 1.19 (6H, t), 2.05 (2H, q), 3.50 (2H, dq), 3.66 (2H, dq), 4.36 (2H, t), 4.66 (1H, t), 7.35 (2H, d), 8.25 (2H, d);

### Step 2:

### 3,3-Diethoxypropyl (1S,2R)-3-[(1,3-benzodioxol-5-ylsulfonyl) (isobutyl) amino] -1- [4-(benzyloxy)benzyl] -2-hydroxypropylcarbamate (328)

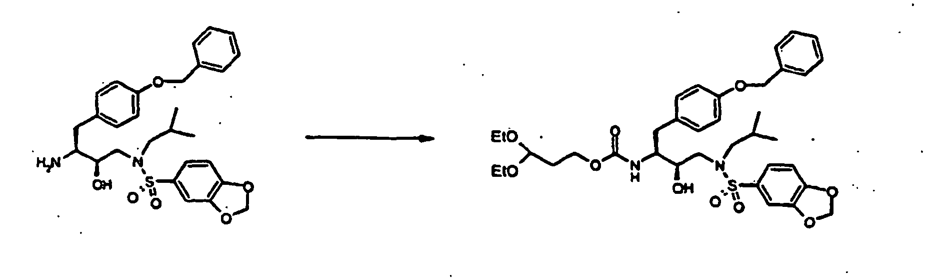

Treatment of *N*-{(2*R*,3*S*)-3-amino-4-[4-(benzyloxy)phenyl]-2-hydroxybutyl}-*N*-isobutyl-1,3-benzodioxole-5-sulfonamide with 3,3-diethoxypropan-1-yl-4'-nitrophenyl carbonate as described above provided the title compound as a white foam in 51% yield. ¹H NMR (DMSO-d₆) : δ 0.78 (6H, dd), 1.33 (6H, dt), 1.65 (2H,q), 1.92 (1H, m), 2.46 (1H, t), 2.69-3.02 (4H, m), 3.27-3.58 (7H, m), 3.80 (2H, t), 4.43 (1H, t), 4.94 (1H, d), 4.99 (2H, s), 6.12 (2H, s), 6.83 (2H, d), 6.97 (1H, d), 7.03 (1H, d), 7.07 (2H, d), 7.23-7.39 (7H, m); MS: 701 (MH⁺)

### Example 112

### Step 1:

### tert-Butyl (1S,2R)-3-[(1,3-benzodioxol-5-ylsulfonyl) (1-ethylpropoxy) amino]-1-[4-(benzyloxy)benzyl]-2-hydroxypropylcarbamate

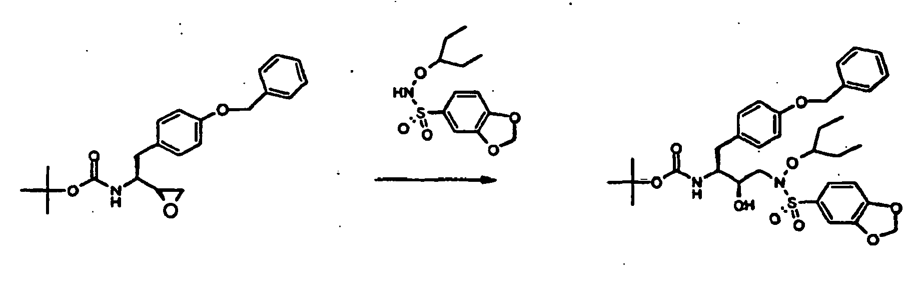

To a solution of *tert*-butyl (1*S*)-2-[4-(benzyloxy)phenyl]-1-[(2*S*)-oxiranyl]ethylcarbamate (2.66 g, 7.2 mmol) and the *N*-(1-ethylpropoxy)-1,3-benzodioxole-5-sulfonamide (2.30 g, 9.0 mmol) in tetrahydrofuran (12 mL) was added phosphazene base P4 *tert*-butyl solution (1.44 mL, 1.44 mmol, 1M in hexane). After stirring at ambient temperature for 18 h, the reaction was concentrated *in vacuo,* taken up in ethyl acetate (100 mL), washed with 0.5 N hydrochloric acid (2x40 mL) followed by water (40 mL), saturated sodium bicarbonate (40 mL) and brine (40 mL), dried (magnesium sulfate) and concentrated. The residue was purified by silica gel chromatography (20% ethyl acetate in hexane) to afford the title compound (4.25 g, 90%) as a white foam. ¹H NMR (DMSO-*d*₆): δ 0.79 (6H, dt), 1.11-1.74 (4H, m), 1.17 (9H, s), 2.37 (1H, t), 2.60-3.02 (3H, m), 3.38-3.49 (1H, m), 3.50-3.61 (1H, m), 4.03 (1H, m), 5.00 (2H, s), 5.04 (1H, d), 6.15 (2H, s), 6.60 (1H, d), 7.03 (2H, d), 7.10 (1H, d), 7.17-7.38 (9H, m)

### Step 2:

### N-{(2R,3S)-3-Amino-4-[4-(benzyloxy)phenyl] -2-hydroxybutyl}-N-(1-ethylpropoxy)-1,3-benzodioxole-5-sulfonamide

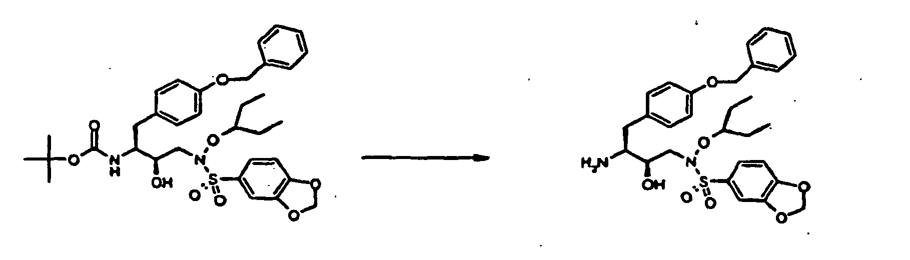

To an ice cold solution of *tert*-Butyl (1*S*,2*R*)-3-[(1,3-benzodioxol-5-ylsulfonyl) (1-ethylpropoxy)amino]-1-[4-(benzyloxy)benzyl]-2-hydroxypropylcarbamate (1.5 g, 2.3 mmol) in dichloromethane (15 mL) was added trifluoroacetic acid (10 mL). After stirring at 5 °C for 3 h, the reaction was concentrated *in vacuo*, taken up in ethyl acetate (80 mL), washed with saturated sodium bicarbonate (2x50 mL), dried (magnesium sulfate) and concentrated *in vacuo* to afford the title compound (1.27 g, quant.) as an off-white foam. ¹H NMR (DMSO-*d*₆) : δ 0.77 (6H, dt), 1.07-1.72 (4H, m), 1.82 (2H, br), 2.28 (1H, t), 2.58-3.05 (4H, m), 3.43-3.53 (1H, m), 3.95-4.03 (1H, m), 4.86 (1H, br s), 5.01 (2H, s), 6.16 (2H, s), 6,86 (2H, d), 7.03 (2H, d), 7.12-7.40 (8H, m);

### Step 3:

### (3R,3aS,6aR)-Hexahydrofuro[2,3-b]furan-3-yl (1S,2R)-3-[(1,3-benzodioxol-5-ylsulfonyl) (1-ethylpropoxy)amino]-1-[4-(benzyloxy)benzyl]-2-hydroxypropylcarbamate (329) not encompassed in the present invention

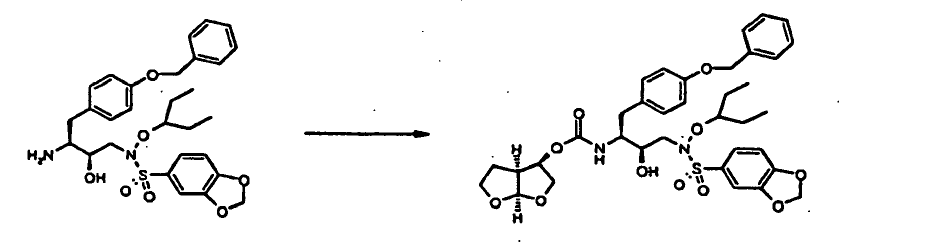

Treatment of *N*-{(2*R*,3*S*)-3-Amino-4-[4-(benzyloxy)phenyl]-2-hydroxybutyl}-*N*-(1-ethylpropoxy)-1,3-benzodioxole-5-sulfonamide with [(3*R*,3a*S*,6a*R*)hexahydrofuro[2,3-*b*]furan-3-yl][4-nitrophenyl] carbonate as described above provided the title compound as a white foam in 66% yield. ¹H NMR (DMSO-*d*₆): δ 0.82 (6H, t), 1.11-1.74 (6H, m), 2.32 (1H, t), 2.69-2.94 (4H, m), 3.45-3.66 (5H, m), 3.75-3.79 (1H, m), 3.99-4.03 (1H, m), 4.78 (1H, q), 5.00 (2H, s), 5.16 (1H, d), 5.46 (1H, d), 6.16 (2H, s), 6.82 (2H, d), 7.03 (2H, d), 7.11-7.38 (9H, m); MS: 713 (MH⁺);
**C₃₆H₄₄N₂O₁₁S.**

### Example 183

### Anti-Viral Activity

We measured the enzyme inhibition constants of the compounds listed in Table I against HIV-1 protease using the methods of: B. Maschera et al., "Human Immunodeficiency Virus: Mutations in the Viral Protease that Confer Resistance to Saquinavir Increase the Dissociation Rate Constant for the Protease-Saquinavir Complex", J. Biol. Chem., 271, pp. 33231-35 (1996); and M. V. Toth et al., Int. J. Peptide Protein Res. 36, pp. 544-50 (1990)

### Antiviral activity assay in MT4 cells

Antiviral HIV activity and compound-induced cytotoxicity were measured in parallel by means of a propidium iodide based procedure in the human T-cell lymphotropic virus transformed cell line MT4. Aliquots of the test compounds were serially diluted in medium (RPMI 1640, 10% fetal calf serum (FCS), and gentamycin) in 96-well plates (Costar 3598) using a Cetus Pro/Pette. Exponentially growing MT4 cells were harvested and centrifuged at 1000 rpm for 10 min in a Jouan centrifuge (model CR 4 12). Cell pellets were resuspended in fresh medium (RPMI 1640, 20% FCS, 20% IL-2, and gentamycin) to a density of 5 x 10⁵ cells/ml. Cell aliquots were infected by the addition of HIV-1 (strain IIIB) diluted to give a viral multiplicity of infection of 100 x TCID₅₀. A similar cell aliquot was diluted with medium to provide a mock-infected control. Cell infection was allowed to proceed for 1 hr at 37° in a tissue culture incubator with humidified 5% CO₂ atmosphere. After the 1 hr incubation the virus/cell suspensions were diluted 6-fold with fresh medium, and 125 µl of the cell suspension was added to each well of the plate containing prediluted compound. Plates were then placed in a tissue culture incubator with humidified 5% CO₂ for 5 days. At the end of the incubation period, 27 µl of 5% Nonidet-40 was added to each well of the incubation plate. After thorough mixing with a Costar multitip pipetter, 60 µl of the mixture was transferred to filter-bottomed 96-well plates. The plates were analyzed in an automated assay instrument (Screen Machine, Idexx Laboratories). The assay makes use of a propidium iodide dye to estimate the DNA content of each well.

### REFERENCES

1. Averett, D.R. 1989. Anti-HIV compound assessment by two novel high capacity assays. J. virol. Methods 23: 263-276.
2. Schwartz, O., et al. 1988. A rapid and simple colorimetric test for the study of anti-HIV agents. AIDS Res. and Human Retroviruses, 4(6):441-447.
3. Daluge, S.M., et al. 1994. 5-chloro-2'3'-deoxy-3'fluorouridine (935U83), a selective anti-human immuno-deficiency virus agent with an improved metabolic and toxicological profile. Antimicro. Agents and Chemother., 38 (7):1590-1603.

The anti-viral potency in MT-4 cells of the compounds set forth in Tables 1 and 2 was determined using the above technique. The results are shown in Table A.

**Table A. Antiviral Activity of Compounds of the Invention.**

| *Cmpd* # | *IC₅₀* |
|---|---|
| 5a | NA |
| 5b | NA |
| 5c | NA |
| 5d | NA |
| 5e | NA |
| 5f | NA |
| 5g | NA |
| 10 | D |
| 11 | D |
| 12 | E |
| 13 | D |
| 14 | NA |
| 15 | D |
| 16 | D |
| 17 | D |
| 18 | C |
| 19 | E |
| 20 | C |
| 21 | C |

In Table A, the following classifications have been employed:
A < 0.001 µM
0.010 > B > 0.001 µM
0.100 > C > 0.010 µM
D > 0.1 µM
"NA" = compound was not tested.

### Anti-viral activity against Resistant Viruses

EP13 and D545701, two multi protease inhibitor resistant viruses were used to assess potency against mutant viruses. These viruses contain the following mutations relative to the consensus sequence of wild type virus:

D545701-14: Protease amino acid sequence: L10I, L19Q, K20R, E35D, M36I, S37N, M46I, I50V, I54V, I62V, L63P, A71V, V82A, L90M; reverse transcriptase amino acid sequence: E28K, K32E, V35I, T39S/T, E40D/V/Y/F, M41M/L, K43E, Y181Y/C

EP13: Protease amino acid sequence: M46I, L63P, A71V, V82F/L, I84V; No reverse transcriptase mutations.

Reference data for the following protease inhibitors are (D545701-14; EP13):
Amprenavir^{™}: >1000nM; 600nM
Indinavir^{™}: 700nM; 560nM
Nelfinavir^{™}: 690nM; N/A
Ritonavir^{™}: >1000nM; >600nM
Saquinavir^{™}: 900nM; N/A

Assays of the above mutant viruses were carried out as described above for the wild type virus and the results are shown below in Table B.

**Table B**

| Compound No. | Wildtype virus - *IC₅₀* | EP13 mutant *IC₅₀* | D545701-14 mutant - *IC₅₀* |
|---|---|---|---|
| 328 | C | NA | NA |

In Table B, the following classifications have been employed:
A < 0.001 µM
0.010 > B > 0.001 µM
0.100 > C > 0.010 µM
D > 0.1 µM
"NA" = compound was not tested.

## Claims

1. A compound selected from compound numbers: 5a, 5b, 5c, 5d, 5e, 5f, 5g, 10-21, 58, 67, 68 and 328, wherein said compound has the structure: 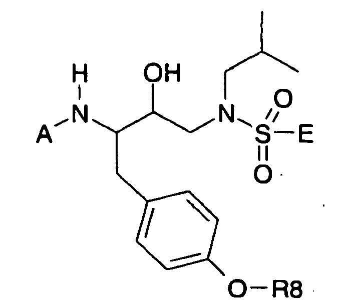
| | A | S(O)₂E | R⁸ |
|---|---|---|---|
| 5a | 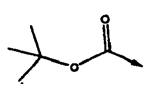 | 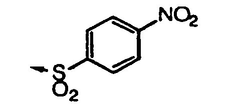 | 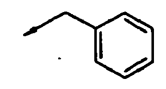 |
| 5b | 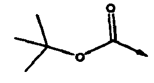 | 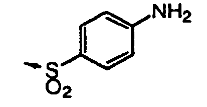 | 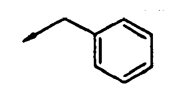 |
| 5c | 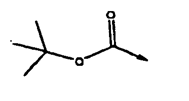 | 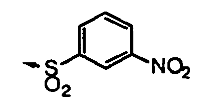 | 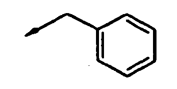 |
| 5d | 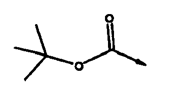 | 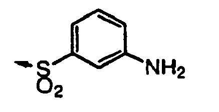 | 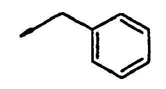 |
| 5e | 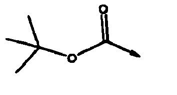 | 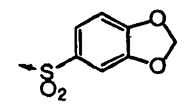 | 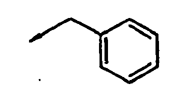 |
| 5f | 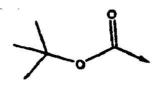 | 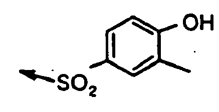 | 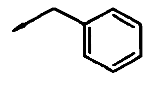 |
| 5g | 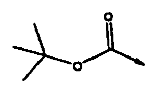 | 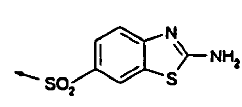 | 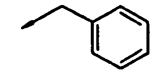 |
| 10 | 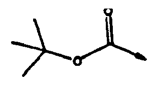 | 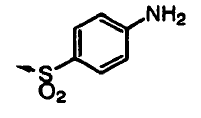 | 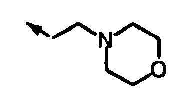 |
| 11 | 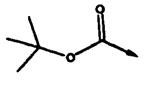 | 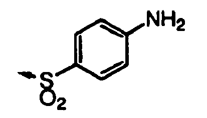 | 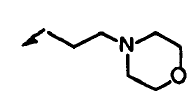 |
| 12 | 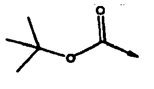 | 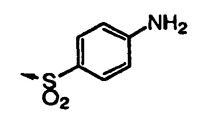 | 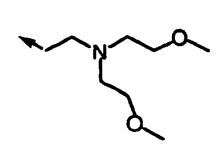 |
| 13 | 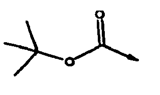 | 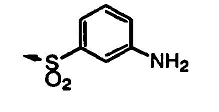 | 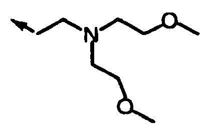 |
| 14 | 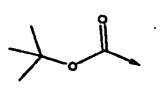 | 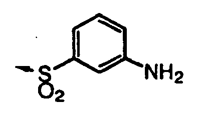 | 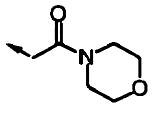 |
| 15 | 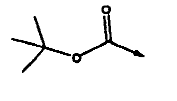 | 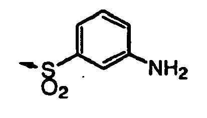 | 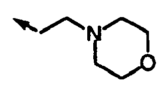 |
| 16 | 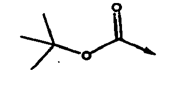 | 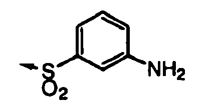 | 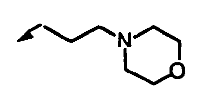 |
| 17 | 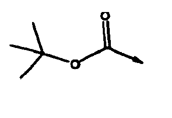 | 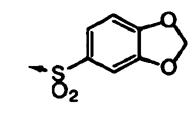 | 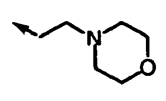 |
| 18 | 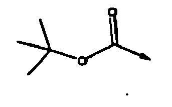 | 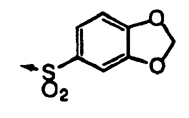 | 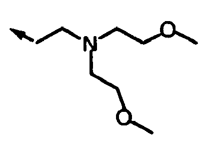 |
| 19 | 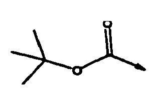 | 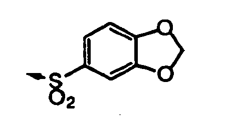 | 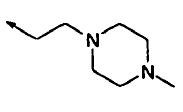 |
| 20 | 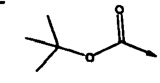 | 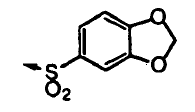 | 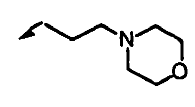 |
| 21 | 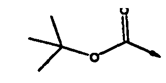 | 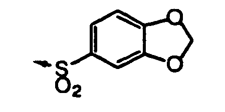 | 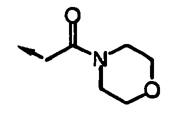 |
| 58 | 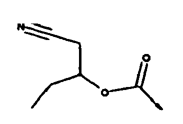 | 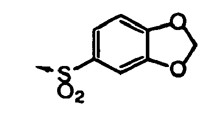 | 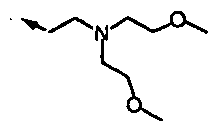 |

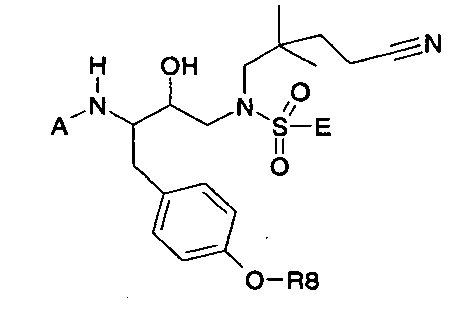
| | A | S(O)₂E | R⁸ |
|---|---|---|---|
| 67 | 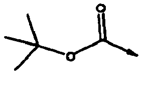 | 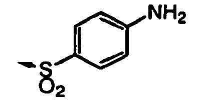 | 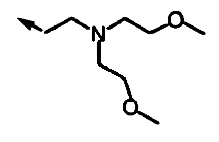 |
| 68 | 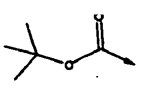 | 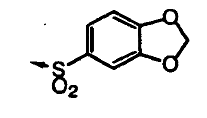 | 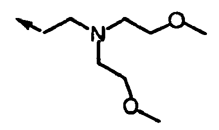 |

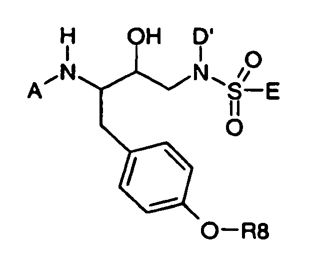
| | A | R⁸ | D' | E |
|---|---|---|---|---|
| 328 | 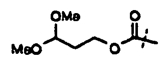 | 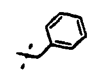 | 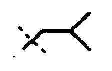 | 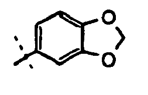 |

2. A pharmaceutical composition comprising a compound according to claim 1, in an amount sufficient to inhibit an aspartyl protease; and a pharmaceutically acceptable carrier.

3. The pharmaceutical composition according to claim 2, wherein said composition is in a pharmaceutically acceptable form for administration to a human being.

4. The pharmaceutical composition according to claim 2, wherein said composition additionally comprises an additional anti-viral agent.

5. The pharmaceutical composition according to claim 2, wherein said composition comprises at least one additional therapeutic agent selected from (1 alpha, 2 beta, 3 alpha)-9-[2,3-bis(hydroxymethyl)cyclobutyl]- guanine [(-)BHCG, SQ-34514]; oxetanocin-G (3,4-bis-(hydroxymethyl)-2-oxetanosylguanine); acyclic nucleosides, such as acyclovir, valaciclovir, famciclovir, ganciclovir or penciclovir; acyclic nucleoside phosphonates, such as (S)-1-(3-hydroxy-2-phosphonyl-methoxypropyl)cytosine (HPMPC); ribonucleotide reductase inhibitors, such as 2-acetylpyridine 5-[(2-chloroanilino)thiocarbonyl) thiocarbonohydrazone, 3'azido-3'-deoxythymidine; other 2',3'-dideoxynucleosides such as 2',3'-dideoxycytidine, 2',3'-dideoxyadenosine, 2',3'-dideoxyinosine, or 2',3'-didehydrothymidine; other aspartyl protease inhibitors, such as indinavir, ritonavir, nelfinavir, or [3S-[3R*(1R*, 2S*)]]-[3[[(4-aminophenyl)sulfonyl](2-methylpropyl)amino]-2-hydroxy-1-(phenylmethyl)propyl]-tetrahydro-3-furanyl ester (amprenavir); oxathiolane nucleoside analogues, such as (-)-cis-1-(2-hydroxymethyl)-1,3-oxathiolane 5-yl)-cytosine (lamivudine) or cis-1-(2-(hydroxymethyl)-1,3-oxathiolan-5-yl)-5-fluorocytosine (FTC); 3'-deoxy-3'-fluorothymidine; 5-chloro-2',3'-dideoxy-3'-fluorouridine; (-)-cis-4-[2-amino-6-(cyclopropylamino)-9H-purin-9-yl]-2-cyclopentene-1-methanol; ribavirin; 9-[4-hydroxy-2-(hydroxymethyl)but-1-yl]-guanine (H2G); tat inhibitors, such as 7-chloro-5-(2-pyrryl)-3H-1,4-benzodiazepin-2-(H)-one (Ro5-3335) or 7-chloro-1,3-dihydro-5-(1H-pyrrol-2-yl)-3H-1,4-benzodiazepin-2-amine (Ro24-7429); interferons, such as α-interferon; renal excretion inhibitors such as probenecid; nucleoside transport inhibitors such as dipyridamole; pentoxifylline; N-acetylcysteine (NAC); Procysteine; α - trichosanthin; phosphonoformic acid; immunomodulators, such as interleukin Π or thymosin; granulocyte macrophage colony stimulating factors; erythropoetin; soluble CD₄ and genetically engineered derivatives thereof; non-nucleoside reverse transcriptase inhibitors (NNRTIs), such as nevirapine (BI-RG-587), loviride (α -APA) or delavuridine (BHAP); 1,4-dihydro-2H-3,1-benzoxazin-2-ones NNRTIs, such as (-)-6-chloro-4-cyclopropylethynyl-4-trifluoromethyl-1,4-dihydro-2H-3,1-benzoxazin-2-one (L-743,726 or DMP-266); or quinoxaline NNRTIs, such as isopropyl (2S)-7-fluoro-3,4-dihydro-2-ethyl-3-oxo-1-(2H)-quinoxalinecarboxylate (HBY1293).

6. The pharmaceutical composition according to any one of claims 2-5, wherein said composition is in an orally available dosage form.

7. The pharmaceutical composition of claim 6 for use in treating a patient infected with a virus that depends upon an aspartyl protease for an obligatory event in its life cycle.

8. The pharmaceutical composition of claim 6 for use in treating a patient infected with HTV-I or HIV-II.

9. The pharmaceutical composition according to claim 7 or 8, wherein an additional therapeutic agent selected from (1 alpha, 2 beta, 3 alpha)-9-[2,3-bis(hydroxymethyl) cyclobutyl]guanine [(-) BHCG, SQ-34514]; oxetanocin-G (3,4-bis-(hydroxymethyl)-2-oxetanosyl]guanine); acyclic nucleosides, such as acyclovir, valaciclovir, famciclovir, ganciclovir or penciclovir; acyclic nucleoside phosphonates, such as (S)-1-(3-hydroxy-2-phosphonyl-methoxypropyl)cytosine (HPMPC); ribonucleotide reductase inhibitors, such as 2-acetylpyridine 5-[(2-chloroanilino)thiocarbonyl) thiocarbonohydrazone, 3'azido-3'-deoxythymidine; other 2',3'-dideoxynucleosides such as 2',3'-dideoxycytidine, 2',3'-dideoxyadenosine, 2',3'-dideoxyinosine, or 2',3'-didehydrothymidine; other aspartyl protease inhibitors, such as indinavir, ritonavir, nelfinavir, or [3S-[3R*(1R*, 2S*)]]-[3[[(4-aminophenyl)sulfonyl](2-methylpropyl)amino]-2-hydroxy-1-(phenylmethyl)propyl]-tetrahydro-3-furanyl ester (amprenavir); oxathiolane nucleoside analogues, such as (-)-cis-1-(2-hydroxymethyl)-1,3-oxathiolane 5-yl)-cytosine (lamivudine) or cis-1-(2-(hydroxymethyl)-1,3-oxathiolan-5-yl)-5-fluorocytosine (FTC); 3'-deoxy-3'-fluorothymidine; 5-chloro-2',3'-dideoxy-3'-fluorouridine; (-)-cis-4-[2-amino-6-(cyclopropylamino)-9H-purin-9-yl]-2-cyclopentene-1-methanol; ribavirin; 9-[4-hydroxy-2-(hydroxymethyl)but-1-yl]-guanine (H2G); tat inhibitors, such as 7-chloro-5-(2-pyrryl)-3H-1,4-benzodiazepin-2-(H)-one (Ro5-3335) or 7-chloro-1,3-dihydro-5-(1H-pyrrol-2-yl)-3H-1,4-benzodiazepin-2-amine (Ro24-7429); interferons, such as α -interferon; renal excretion inhibitors such as probenecid; nucleoside transport inhibitors such as dipyridamole; pentoxifylline; N-acetylcysteine (NAC); Procysteine; α-trichosanthin; phosphonoformic acid; immunomodulators, such as interleukin II or thymosin; granulocyte macrophage colony stimulating factors; erythropoetin; soluble CD₄ and genetically engineered derivatives thereof; non-nucleoside reverse transcriptase inhibitors (NNRTIs), such as nevirapine (BI-RG-587), Ioviride (α-APA) or delavuridine (BHAP); 1,4-dihydro-2H-3,1-benzoxazin-2-ones NNRTIs, such as (-)-6-chloro-4-cyclopropylethynyl-4-trifluoromethyl-1,4-dihydro-2H-3,1-benzoxazin-2-one (L-743,726 or DMP-266); or quinoxaline NNRTIs, such as isopropyl (2S)-7-fluoro-3,4-dihydro-2-ethyl-3-oxo-1-(2H)-quinoxalinecarboxylate (HBY1293), is to be administered as either a separate dosage form or as a single dosage form together with said compound.

10. The pharmaceutical composition according to claim 6 for use in treating a patient diagnosed with AIDS; AIDS related complex (ARC); progressive generalized lymphadenopathy (PGL); Kaposi's sarcoma, thrombocytopenic purpura; AIDS-related neurological conditions such as AIDS dementia complex, multiple sclerosis or tropical paraperesis; anti-HIV antibody-positive conditions; or HIV-positive conditions.

11. The pharmaceutical composition according to claim 10, wherein an additional therapeutic agent selected from (1 alpha, 2 beta, 3 alpha)-9-[2,3-bis(hydroxymethyl) cyclobutyl]guanine [(-) BHCG, SQ-34514]; oxetanocin-G (3,4-bis-(hydroxymethyl)-2-oxetanosyl]guanine); acyclic nucleosides, such as acyclovir, valaciclovir, famciclovir, ganciclovir or penciclovir, acyclic nucleoside phosphonates, such as (S)-1-(3-hydroxy-2-phosphonyl-methoxypropyl)cytosine (HPMPC); ribonucleotide reductase inhibitors, such as 2-acetylpyridine 5-[(2-chloroanilino)thiocarbonyl) thiocarbonohydrazone, 3'azido-3'-deoxythymidine; other 2',3'-dideoxynucleosides such as 2',3'-dideoxycytidine, 2',3'-dideoxyadenosine, 2',3'-dideoxyinosine, or 2',3'-didehydrothymidine; other aspartyl protease inhibitors, such as indinavir, ritonavir, nelfinavir, or [3S-[3R*(1R*, 2S*)]]-[3[[(4-aminophenyl)sulfonyl](2-methylpropyl)amino]-2-hydroxy-1-(phenylmethyl)propyl]-tetrahydro-3-furanyl ester (amprenavir); oxathiolane nucleoside analogues, such as (-)-cis-1-(2-hydroxymethyl)-1,3-oxathiolane 5-yl)-cytosine (lamivudine) or cis-1-(2-(hydroxymethyl)-1,3-oxathiolan-5-yl)-5-fluorocytosine (FTC); 3'-deoxy-3'-fluorothymidine; 5-chloro-2',3'-dideoxy-3'-fluorouridine; (-)-cis-4-[2-amino-6-(cyclopropylamino)-9H-purin-9-yl]-2-cyclopentene-1-methanol; ribavirin; 9-[4-hydroxy-2-(hydroxymethyl)but-1-yl]-guanine (H2G); tat inhibitors, such as 7-chloro-5-(2-pyrryl)-3H-1,4-benzodiazepin-2-(H)-one (Ro5-3335) or 7-chloro-1,3-dihydro-5-(1H-pyrrol-2-yl)-3H-1,4-benzodiazepin-2-amine (Ro24-7429); interferons, such as α-interferon; renal excretion inhibitors such as probenecid; nucleoside transport inhibitors such as dipyridamole; pentoxifylline; N-acetylcysteine (NAC); Procysteine; α-trichosanthin; phosphonoformic acid; immunomodulators, such as interleukin II or thymosin; granulocyte macrophage colony stimulating factors; erythropoetin; soluble CD₄ and genetically engineered derivatives thereof; non-nucleoside reverse transcriptase inhibitors (NNRTIs), such as nevirapine (BI-RG-587), loviride (α -APA) or delavuridine (BHAP); 1,4-dihydro-2H-3,1-benzoxazin-2-ones NNRTIs, such as (-)-6-chloro-4-cyclopropylethynyl-4-trifluoromethyl-1,4-dihydro-2H-3,1-benzoxazin-2-one (L-743,726 or DMP-266); or quinoxaline NNRTIs, such as isopropyl (2S)-7-fluoro-3,4-dihydro-2-ethyl-3-oxo-1-2H)-quinoxalinecarboxylate (HBY1293), is to be administered as either a separate dosage form or as a single dosage form together with said compound.

## Patentansprüche

1. Verbindung, ausgewählt aus Verbindungsnummern: 5a, 5b, 5c, 5d, 5e, 5f, 5g, 10-21, 58, 67, 68 und 328, wobei die Verbindung die Struktur aufweist: 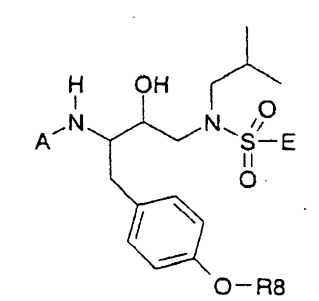
| | A | S(O)₂E | R⁸ |
|---|---|---|---|
| 5a | 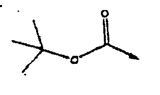 | 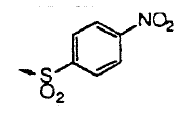 | 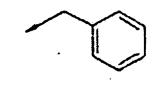 |
| 5b | 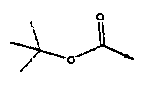 | 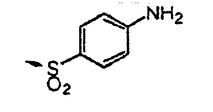 | 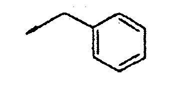 |
| 5c | 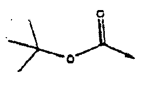 | 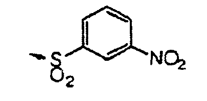 | 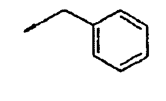 |
| 5d | 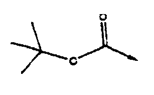 | 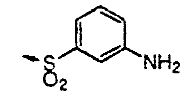 | 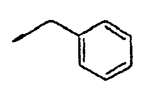 |
| 5e | 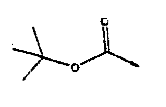 | 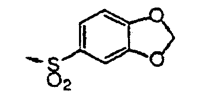 | 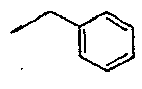 |
| 5f | 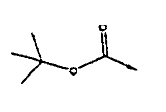 | 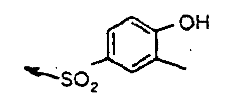 | 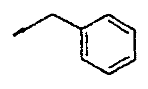 |
| 5g | 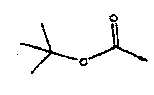 | 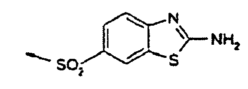 | 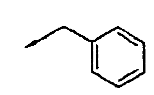 |
| 10 | 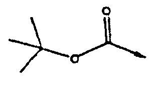 | 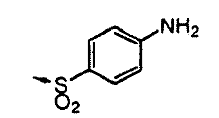 | 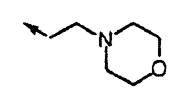 |
| 11 | 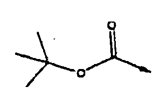 | 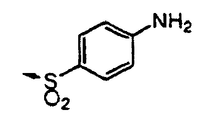 | 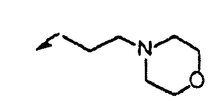 |
| 12 | 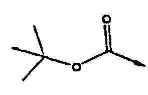 | 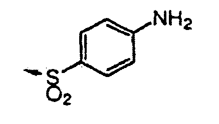 | 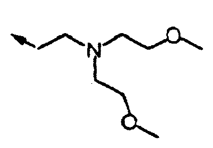 |
| 13 | 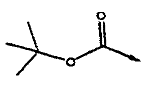 | 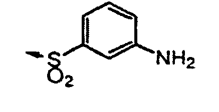 | 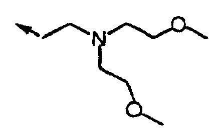 |
| 14 | 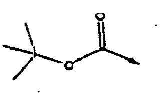 | 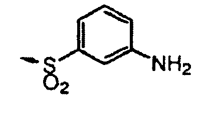 | 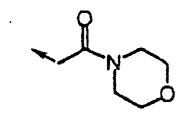 |
| 15 | 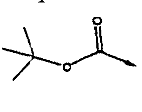 | 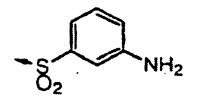 | 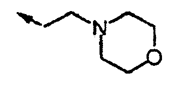 |
| 16 | 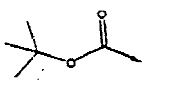 | 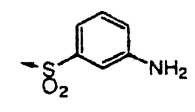 | 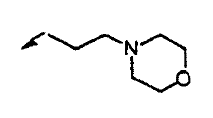 |
| 17 | 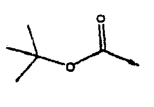 | 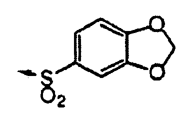 | 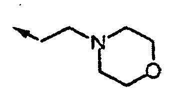 |
| 18 | 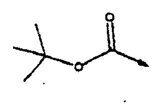 | 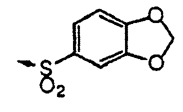 | 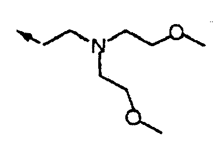 |
| 19 | 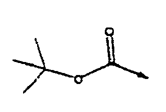 | 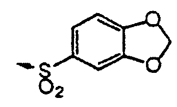 | 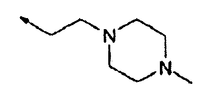 |
| 20 | 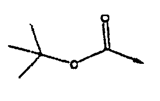 | 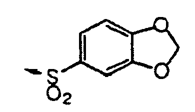 | 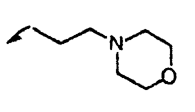 |
| 21 | 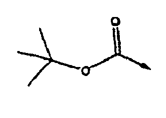 | 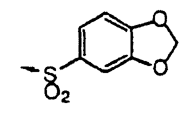 | 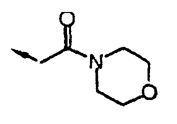 |
| 58 | 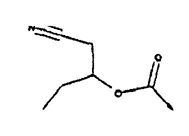 | 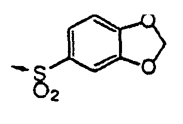 | 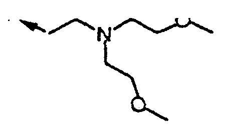 |

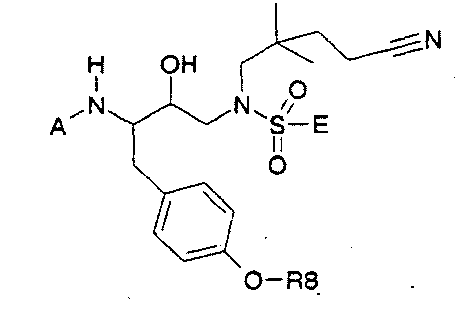
| | A | S(O)₂E | R⁸ |
|---|---|---|---|
| 67 | 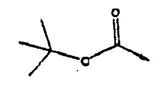 | 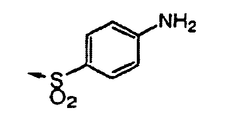 | 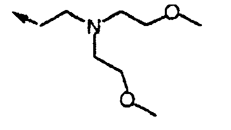 |
| 68 | 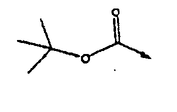 | 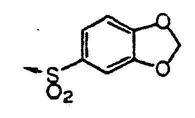 | 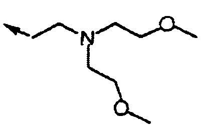 |

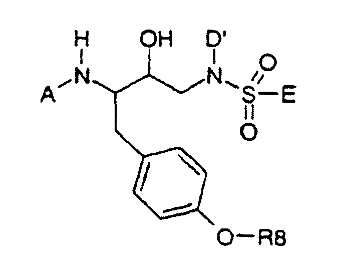
| | A | R⁸ | D' | E |
|---|---|---|---|---|
| 328 | 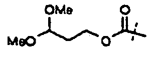 | 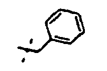 | 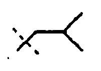 | 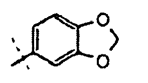 |

2. Arzneimittel, umfassend eine Verbindung nach Anspruch 1, in einer Menge, die ausreicht, um eine Aspartylprotease zu hemmen; und einen pharmazeutisch verträglichen Träger.

3. Arzneimittel nach Anspruch 2, wobei die Zusammensetzung in einer pharmazeutisch verträglichen Form zur Verabreichung an einen Menschen vorliegt.

4. Arzneimittel nach Anspruch 2, wobei die Zusammensetzung ferner ein zusätzliches Antivirusmittel umfasst.

5. Arzneimittel nach Anspruch 2, wobei die Zusammensetzung mindestens ein zusätzliches therapeutisches Mittel umfasst, ausgewählt aus (1-alpha, 2-beta, 3-alpha)-9-[2,3-Bis(hydroxymethyl)cyclobutyl]-guanin [(-)BHCG, SQ-34514]; Oxetanocin-G (3,4-Bis-(hydroxymethyl)-2-oxetanosyl-guanin); acyclischen Nucleosiden, wie Acyclovir, Valaciclovir, Famciclovir, Ganciclovir oder Penciclovir; acyclischen Nucleosidphosphonaten, wie (S)-1-(3-Hydroxy-2-phosphonylmethoxypropyl)cytosin (HPMPC); Ribonucleotidreduktaseinhibitoren, wie 2-Acetylpyridin-5-[(2-chloranilino)thiocarbonyl)thiocarbonhydrazon, 3'-Azido-3'-desoxythymidin; anderen 2',3'-Didesoxynucleosiden, wie 2',3'-Didesoxycytidin, 2',3'-Didesoxyadenosin, 2',3'-Didesoxyinosin oder 2',3'-Didehydrothymidin; anderen Aspartylproteaseinhibitoren, wie Indinavir, Ritonavir, Nelfinavir oder [3S-[3R*(1R*,2S*)]]-[3[[(4-Aminophenyl)sulfonyl](2-methylpropyl)amino]-2-hydroxy-1-(phenylmethyl)propyl]-tetrahydro-3-furanylester (Amprenavir); Oxathiolannucleosidanalogen, wie (-)-cis-1-(2-Hydroxymethyl)-1,3-oxathiolan-5-yl)-cytosin (Lamivudin) oder cis-1-(2-(Hydroxymethyl)-1,3-oxathiolan-5-yl)-5-fluorcytosin (FTC); 3'-Desoxy-3'-fluorthymidin; 5-Chlor-2',3'-didesoxy-3'-fluoruridin; (-)-cis-4-[2-Amino-6-(cyclopropylamino)-9H-purin-9-yl]-2-cyclopenten-1-methanol; Ribavirin; 9-[4-Hydroxy-2-(hydroxymethyl)but-1-yl]-guanin (H2G); Tat-Inhibitoren, wie 7-Chlor-5-(2-pyrryl)-3H-1,4-benzodiazepin-2-(H)-on (Ro5-3335) oder 7-Chlor-1,3-dihydro-5-(1H-pyrrol-2-yl)-3H-1,4-benzodiazepin-2-amin (Ro24-7429); Interferonen, wie α-Interferon; Inhibitoren der Nierenausscheidung, wie Probenecid; Nucleosidtransport-Inhibitoren, wie Dipyridamol; Pentoxifyllin; N-Acetylcystein (NAC); Procystein; α-Trichosanthin; Phosphonoameisensäure; Immunmodulatoren, wie Interleukin II oder Thymosin; Granulocyten-Makrophagen-Kolonie-stimulierenden Faktoren; Erythropoetin; löslichem CD₄ und genetisch manipulierten Derivaten davon; Nichtnucleosid-Reverse-Transcriptase-Inhibitoren (NNRTIs), wie Nevirapin (BI-RG-587), Lovirid (α-APA) oder Delavuridin (BHAP); 1,4-Dihydro-2H-3,1-benzoxazin-2-onen NNRTIs, wie (-)-6-Chlor-4-cyclopropylethinyl-4-trifluormethyl-1,4-dihydro-2H-3,1-benzoxazin-2-on (L-743,726 oder DMP=266); oder Chinoxalin NNRTIs, wie Isopropyl-(2S)-7-fluor-3,4-dihydro-2-ethyl-3-oxo-1(2H)-chinoxalincarboxylat (HBY1293).

6. Arzneimittel nach einem der Ansprüche 2-5, wobei die Zusammensetzung in einer oral verwendbaren Dosierungsform vorliegt.

7. Arzneimittel nach Anspruch 6 zur Verwendung bei der Behandlung eines Patienten, der mit einem Virus infiziert ist, das für ein obligatorisches Ereignis in seinem Lebenszyklus von einer Aspartylprotease abhängig ist.

8. Arzneimittel nach Anspruch 6 zur Verwendung bei der Behandlung eines Patienten, der mit HIV-I oder HIV-II infiziert ist.

9. Arzneimittel nach Anspruch 7 oder 8, wobei ein zusätzliches therapeutisches Mittel, ausgewählt aus (1-alpha, 2-beta, 3-alpha)-9-[2,3-Bis(hydroxymethyl)cyclobutyl]-guanin [(-)BHCG, SQ-34514]; Oxetanocin-G (3,4-Bis-(hydroxymethyl)-2-oxetanosyl]guanin); acyclischen Nucleosiden, wie Acyclovir, Valaciclovir, Famciclovir, Ganciclovir oder Penciclovir; acyclischen Nucleosidphosphonaten, wie (S)-1-(3-Hydroxy-2-phosphonylmethoxypropyl)cytosin (HPMPC); Ribonucleotidreduktaseinhibitoren, wie 2-Acetylpyridin-5-[(2-chloranilino)thiocarbonyl)thiocarbonhydrazon, 3'-Azido-3'-desoxythymidin; anderen 2',3'-Didesoxynucleosiden, wie 2',3'-Didesoxycytidin, 2',3'-Didesoxyadenosin, 2',3'-Didesoxyinosin oder 2',3'-Didehydrothymidin; anderen Aspartylproteaseinhibitoren, wie Indinavir, Ritonavir, Nelfinavir oder [3S-[3R*(1R* ,2S*)]]-[3[[(4-Aminophenyl)sulfonyl](2-methylpropyl)amino]-2-hydroxy-1-(phenylmethyl)propyl]-tetrahydro-3-furanylester(Amprenavir); Oxathiolannucleosidanalogen, wie (-)-cis-1-(2-Hydroxymethyl)-1,3-oxathiolan-5-yl)-cytosin (Lamivudin) oder cis-1-(2-(Hydroxymethyl)-1,3-oxathiolan-5-yl)-5-fluorcytosin (FTC); 3'-Desoxy-3'-fluorthymidin; 5-Chlor-2',3'-didesoxy-3'-fluoruridin; (-)-cis-4-[2-Amino-6-(cyclopropylamino)-9H-purin-9-yl]-2-cyclopenten-1-methanol; Ribavirin; 9-[4-Hydroxy-2-(hydroxymethyl)but-1-yl]-guanin (H2G); Tat-Inhibitoren, wie 7-Chlor-5-(2-pyrryl)-3H-1,4-benzodiazepin-2-(H)-on (Ro5-3335) oder 7-Chlor-1,3-dihydro-5-(1H-pyrrol-2-yl)-3H-1,4-benzodiazepin-2-amin (Ro24-7429); Interferonen, wie α-Interferon; Inhibitoren der Nierenausscheidung, wie Probenecid; Nucleosidtransport-Inhibitoren, wie Dipyridamol; Pentoxifyllin; N-Acetylcystein (NAC); Procystein; α-Trichosanthin; Phosphonoameisensäure; Immunmodulatoren, wie Interleukin II oder Thymosin; Granulocyten-Makrophagen-Kolonie-stimulierenden Faktoren; Erythropoetin; löslichem CD₄ und genetisch manipulierten Derivaten davon; Nichtnucleosid-Reverse-Transcriptase-Inhibitoren (NNRTIs), wie Nevirapin (BI-RG-587), Lovirid (α-APA) oder Delavuridin (BHAP); 1,4-Dihydro-2H-3,1-benzoxazin-2-onen NNRTIs, wie (-)-6-Chlor-4-cyclopropylethinyl-4-trifluormethyl-1,4-dihydro-2H-3,1-benzoxazin-2-on (L-743,726 oder DMP-266); oder Chinoxalin NNRTIs, wie Isopropyl-(2S)-7-fluor-3,4-dihydro-2-ethyl-3-oxo-1-(2H)-chinoxalincarboxylat (HBY1293), entweder als eine separate Dosierungsform oder als eine einzelne Dosierungsform zusammen mit der Verbindung verabreicht werden soll.

10. Arzneimittel nach Anspruch 6 zur Verwendung bei der Behandlung eines Patienten, bei dem AIDS; mit AIDS in Zusammenhang stehende Symptome (AIDS Related Complex - ARC); progressive generalisierte Lymphadenopathie (PGL); Kaposi-Sarkom, thrombocytopenische Purpura; mit AIDS in Zusammenhang stehende neurologische Zustände, wie AIDS-Demenz, Multiple Sklerose oder tropische Paraperese; Anti-HIV-Antikörper-positive Zustände; oder HIV-positive Zustände diagnostiziert wurden.

11. Arzneimittel nach Anspruch 10, wobei ein zusätzliches therapeutisches Mittel, ausgewählt aus (1-alpha, 2-beta, 3-alpha)-9-[2,3-Bis(hydroxymethyl)cyclobutyl]-guanin [(-)BHCG, SQ-34514]; Oxetanocin-G (3,4-Bis-(hydroxymethyl)-2-oxetanosyl]guanin); acyclischen Nucleosiden, wie Acyclovir, Valaciclovir, Famciclovir, Ganciclovir oder Penciclovir; acyclischen Nucleosidphosphonaten, wie (S)-1-(3-Hydroxy-2-phosphonylmethoxypropyl)cytosin (HPMPC); Ribonucleotidreduktaseinhibitoren, wie 2-Acetylpyridin-5-[(2-chloranilino)thiocarbonyl)thiocarbonhydrazon, 3'-Azido-3'-desoxythymidin; anderen 2',3'-Didesoxynucleosiden, wie 2',3'-Didesoxycytidin, 2',3'-Didesoxyadenosin, 2',3'-Didesoxyinosin oder 2',3'-Didehydrothymidin; anderen Aspartylproteaseinhibitoren, wie Indinavir, Ritonavir, Nelfinavir oder [3S-[3R*(1R*,2S*)]]-[3[[(4-Aminophenyl)sulfonyl](2-methylpropyl)amino]-2-hydroxy-1-(phenylmethyl)propyl]-tetrahydro-3-furanylester (Amprenavir); Oxathiolannucleosidanalogen, wie (-)-cis-1-(2-Hydroxymethyl)-1,3-oxathiolan-5-yl)-cytosin (Lamivudin) oder cis-1-(2-(Hydroxymethyl)-1,3-oxathiolan-5-yl)-5-fluorcytosin (FTC); 3'-Desoxy-3'-fluorthymidin; 5-Chlor-2',3'-didesoxy-3'-fluoruridin; (-)-cis-4-[2-Amino-6-(cyclopropylamino)-9H-purin-9-yl]-2-cyclopenten-1-methanol; Ribavirin; 9-[4-Hydroxy-2-(hydroxymethyl)but-1-yl]-guanin (H2G); Tat-Inhibitoren, wie 7-Chlor-5-(2-pyrryl)-3H-1,4-benzodiazepin-2-(H)-on (Ro5-3335) oder 7-Chlor-1,3-dihydro-5-(1H-pyrrol-2yl)-3H-1,4-benzodiazepin-2-amin (Ro24-7429); Interferonen, wie α-Interferon; Inhibitoren der Nierenausscheidung, wie Probenecid; Nucleosidtransport-Inhibitoren, wie Dipyridamol; Pentoxifyllin; N-Acetylcystein (NAC); Procystein; α-Trichosanthin; Phosphonoameisensäure; Immunmodulatoren, wie Interleukin II oder Thymosin; Granulocyten-Makrophagen-Kolonie-stimulierenden-Faktoren; Erythropoetin; löslichem CD₄ und genetisch manipulierten Derivaten davon; Nichtnucleosid-Reverse-Transcriptase-Inhibitoren (NNRTIs), wie Nevirapin (BI-RG-587), Lovirid (α-APA) oder Delavuridin (BHAP); 1,4-Dihydro-2H-3,1-benzoxazin-2-onen NNRTIs, wie (-)-6-Chlor-4-cyclopropylethinyl-4-trifluormethyl-1,4-dihydro-2H-3,1-benzoxazin-2-on (L-743,726 oder DMP-266); oder Chinoxalin NNRTIs, wie Isopropyl-(2S)-7-fluor-3,4-dihydro-2-ethyl-3-oxo-1-(2H)-chinoxalincarboxylat (HBY1293), entweder als eine separate Dosierungsform oder als eine einzelne Dosierungsform zusammen mit der Verbindung verabreicht werden soll.

## Revendications

1. Composé choisi parmi les composés numéros : 5a, 5b, 5c, 5d, 5e, 5f, 5g, 10-21, 58, 67, 68 et 328, dans lequel ledit composé a la structure : 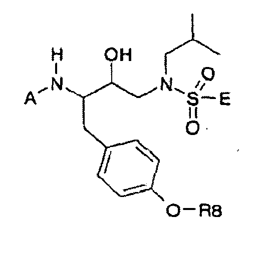
| | A | S(O)₂E | R⁸ |
|---|---|---|---|
| 5a | 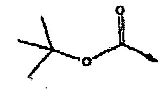 | 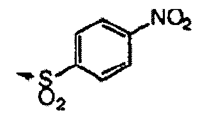 | 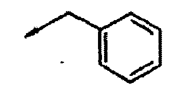 |
| 5b | 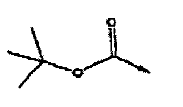 | 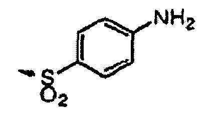 | 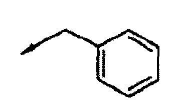 |
| 5c | 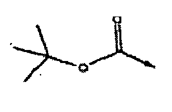 | 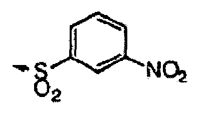 | 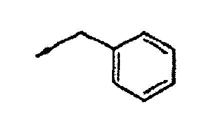 |
| 5d | 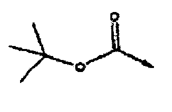 | 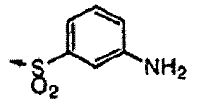 | 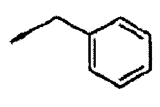 |
| 5e | 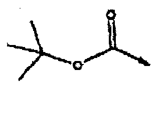 | 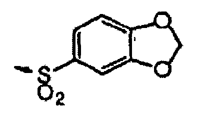 | 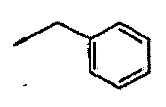 |
| 5f | 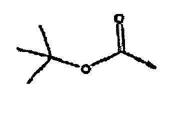 | 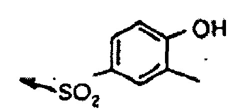 | 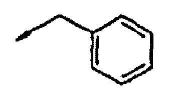 |
| 5g | 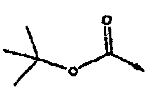 | 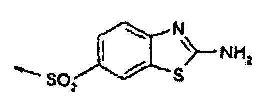 | 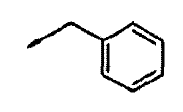 |
| 10 | 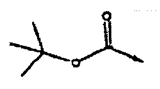 | 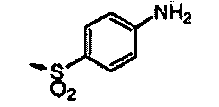 | 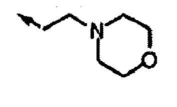 |
| 11 | 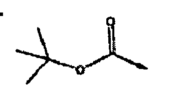 | 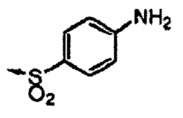 | 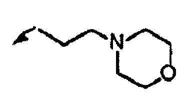 |
| 12 | 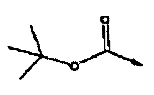 | 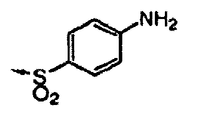 | 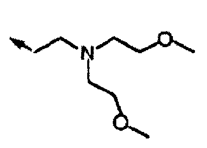 |
| 13 | 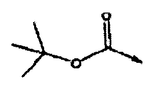 | 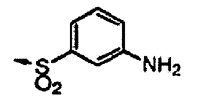 | 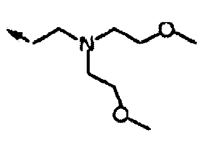 |
| 14 | 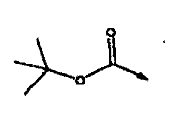 | 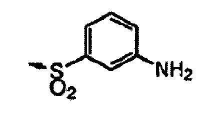 | 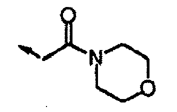 |
| 15 | 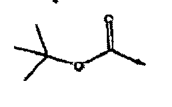 | 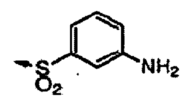 | 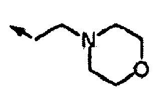 |
| 16 | 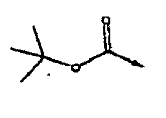 | 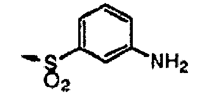 | 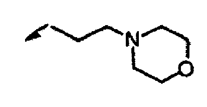 |
| 17 | 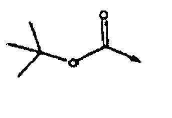 | 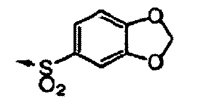 | 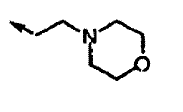 |
| 18 | 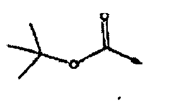 | 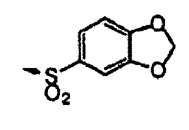 | 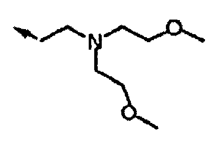 |
| 19 | 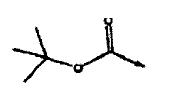 | 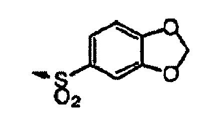 | 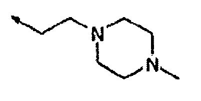 |
| 20 | 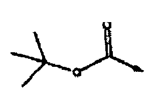 | 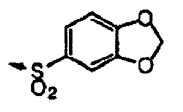 | 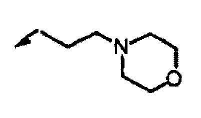 |
| 21 | 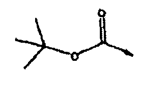 | 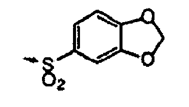 | 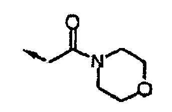 |
| 58 | 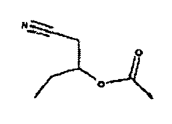 | 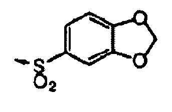 | 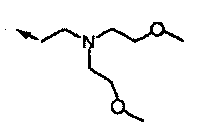 |

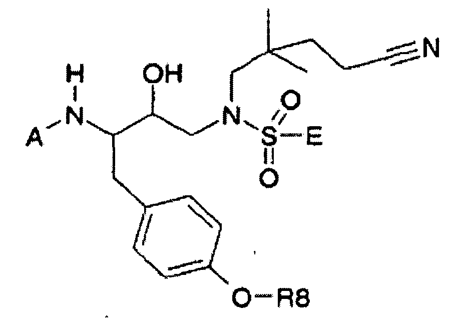
| | A | S(O)₂E | R⁸ |
|---|---|---|---|
| 67 | 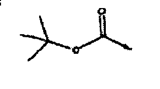 | 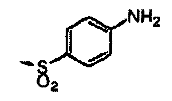 | 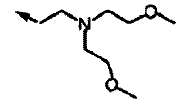 |
| 68 | 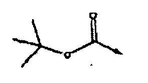 | 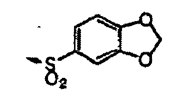 | 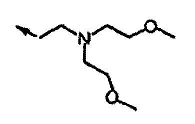 |

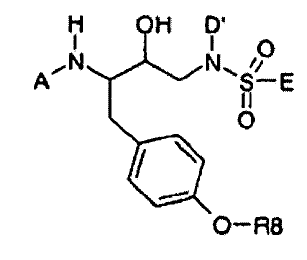
| | A | R⁸ | D' | E |
|---|---|---|---|---|
| 328 | 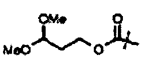 | 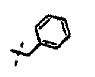 | 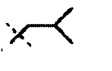 | 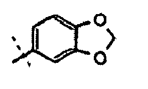 |

2. Composition pharmaceutique comprenant un composé selon la revendication 1, en une quantité suffisante pour inhiber une aspartyl protéase ; et un véhicule pharmaceutiquement acceptable.

3. Composition pharmaceutique selon la revendication 2, dans laquelle ladite composition est sous une forme pharmaceutiquement acceptable pour une administration à un sujet humain.

4. Composition pharmaceutique selon la revendication 2, dans laquelle ladite composition comprend en outre un agent antiviral supplémentaire.

5. Composition pharmaceutique selon la revendication 2, dans laquelle ladite composition comprend au moins un agent thérapeutique supplémentaire choisi parmi la (1-alpha, 2-bêta, 3-alpha)-9-[2,3-bis(hydroxyméthyl)cyclobutyl]-guanine [(-)BHCG, SQ-34514] ; l'oxétanocine-G (3,4-bis-(hydroxyméthyl)-2-oxétanosyl-guanine) ; les nucléosides acycliques, tels que l'acyclovir, le valaciclovir, le famciclovir, le ganciclovir ou le penciclovir ; les phosphonates de nucléosides acycliques, tels que la (S)-1-(3-hydroxy-2-phosphonyl-méthoxypropyl)cytosine (HPMPC) ; les inhibiteurs de ribonucléotide réductase tels que la 5-[(2-chloroanilino)thiocarbonyl]thiocarbonohydrazone de 2-acétylpyridine, la 3'-azido-3'-désoxythymidine ; d'autres 2',3'-didésoxynucléosides tels que la 2',3'-didésoxy-cytidine, la 2',3'-didésoxyadénosine, la 2',3'-didésoxyinosine, ou la 2',3'-didéshydrothymidine ; d'autres inhibiteurs d'aspartyl protéase, tels que l'indinavir, le ritonavir, le nelfinavir, ou l'ester de [3S-[3R*(1R*,2S*)]]-[3[[(4-aminophényl)sulfonyl]-(2-méthylpropyl)amino]-2-hydroxy-1-(phénylméthyl)propyl]-tétrahydro-3-furanyle (amprénavir) ; les analogues de nucléosides oxathiolane, tels que la (-)-cis-1-(2-hydroxyméthyl)-1,3-oxathiolan-5-yl)-cytosine (lamivudine) ou la cis-1-(2-(hydroxyméthyl)-1,3-oxathiolan-5-yl)-5-fluorocytosine (FTC) ; la 3'-désoxy-3'-fluoro-thymidine ; la 5-chloro-2',3'-didésoxy-3'-fluorouridine ; le (-)-cis-4-[2-amino-6-(cyclopropylamino)-9H-purin-9-yl]-2-cyclopentène-1-méthanol ; la ribavirine ; la 9-[4-hydroxy-2-(hydroxyméthyl)but-1-yl]-guanine (H2G) ; les inhibiteurs de tat, tels que la 7-chloro-5-(2-pyrryl)-3H-1,4-benzodiazépin-2-(H) one (Ro5-3335) ou la 7-chloro-1,3-dihydro-5-(1H-pyrrol-2 yl)-3H-1,4-benzodiazépin-2-amine (Ro24-7429) ; les interférons, tels que l'interféron α ; les inhibiteurs d'excrétion rénale tels que le probénécide ; les inhibiteurs du transport des nucléosides tels que le dipyridamole ; la pentoxifylline ; la N-acétylcystéine (NAC) ; la procystéine ; l'α-trichosanthine ; l'acide phosphonoformique ; les immunomodulateurs, tels que l'interleukine II ou la thymosine ; les facteurs de stimulation des colonies granulocytaires/macrophages ; l'érythropoïétine ; les CD₄ solubles et leurs dérivés génétiquement modifiés ; les inhibiteurs de transcriptase inverse non nucléosidiques (NNRTIs), tels que la névirapine (BI-RG-587), le loviride (α-APA) ou la délavuridine (BHAP) ; les NNRTIs de type 1,4-dihydro-2H-3,1-benzoxazin-2-ones tels que la (-)-6-chloro-4-cyclopropyléthyny 1-4-trifluorométhyl-1,4-dihydro-2H-3,1-benzoxazin-2-one (L-743,726 ou DMP-266) ; ou les NNRTIs de type quinoxaline, tels que le (2S)-7-fluoro-3,4-dihydro-2-éthyl-3-oxo-1 (2H)-quinoxalinecarboxylate d'isopropyle (HBY1293).

6. Composition pharmaceutique selon l'une quelconque des revendications 2 à 5, dans laquelle ladite composition est sous une forme pharmaceutique à usage oral.

7. Composition pharmaceutique selon la revendication 6 pour son utilisation dans le traitement d'un patient infecté par un virus qui dépend d'une aspartyl protéase pour un événement obligatoire dans son cycle de vie.

8. Composition pharmaceutique selon la revendication 6 pour son utilisation dans le traitement d'un patient infecté par le VIH-I ou le VIH-II.

9. Composition pharmaceutique selon les revendications 7 ou 8, dans laquelle un agent thérapeutique supplémentaire choisi parmi la (1-alpha, 2-bêta, 3-alpha)-9-[2,3-bis(hydroxyméthyl)cyclobutyl]-guanine [(-)BHCG, SQ-34514] ; l'oxétanocine-G (3,4-bis-(hydroxyméthyl)-2-oxétanosyl]-guanine) ; les nucléosides acycliques, tels que l'acyclovir, le valaciclovir, le famciclovir, le ganciclovir ou le penciclovir ; les phosphonates de nucléosides acycliques, tels que la (S)-1-(3-hydroxy-2-phosphonyl-méthoxypropyl)cytosine (HPMPC) ; les inhibiteurs de ribonucléotide réductase tels que la 5-[(2-chloroanilino)thiocarbonyl]-thiocarbonohydrazone de 2-acétylpyridine, la 3'-azido-3'-désoxythymidine ; d'autres 2',3'-didésoxynucléosides tels que la 2',3'-didésoxycytidine, la 2',3'-didésoxyadénosine, la 2',3'-didésoxyinosine, ou la 2',3'-didéshydrothymidine ; d'autres inhibiteurs d'aspartyl protéase, tels que l'indinavir, le ritonavir, le nelfinavir, ou l'ester de [3S-[3R*(1R*,2S*)]]-[3[[(4-aminophényl)sulfonyl]-(2-méthylpropyl)amino]-2-hydroxy-1-(phénylméthyl)propyl]-tétrahydro-3-furanyle (amprénavir) ; les analogues de nucléosides oxathiolane, tels que la (-)-cis-1-(2-hydroxyméthyl)-1,3-oxathiolan-5-yl)-cytosine (lamivudine) ou la cis-1-(2-(hydroxyméthyl)-1,3-oxathiolan-5-yl)-5-fluorocytosine (FTC) ; la 3'-désoxy-3'-fluoro-thymidine ; la 5-chloro-2',3'-didésoxy-3'-fluorouridine ; le (-)-cis-4-[2-amino-6-(cyclopropylamino)-9H-purin-9-yl]-2-cyclopentène-1-méthanol ; la ribavirine ; la 9-[4-hydroxy-2-(hydroxyméthyl)but-1-yl]-guanine (H2G) ; les inhibiteurs de tat, tels que la 7-chloro-5-(2-pyrryl)-3H-1,4-benzodiazépin-2-(H) one (Ro5-3335) ou la 7-chloro-1,3-dihydro-5-(1H-pyrrol-2 yl)-3H-1,4-benzodiazépin-2-amine (Ro24-7429) ; les interférons, tels que l'interféron α ; les inhibiteurs d'excrétion rénale tels que le probénécide ; les inhibiteurs du transport des nucléosides tels que le dipyridamole ; la pentoxifylline ; la N-acétylcystéine (NAC) ; la procystéine ; l'α-trichosanthine ; l'acide phosphonoformique ; les immunomodulateurs, tels que l'interleukine II ou la thymosine ; les facteurs de stimulation des colonies granulocytaires/macrophages ; l'érythropoïétine ; les CD₄ solubles et leurs dérivés génétiquement modifiés ; les inhibiteurs de transcriptase inverse non nucléosidiques (NNRTIs), tels que la névirapine (BI-RG-587), le loviride (α-APA) ou la délavuridine (BHAP) ; les NNRTI de type 1,4-dihydro-2H-3,1-benzoxazin-2-ones tels que la (-)-6-chloro-4-cyclopropyléthynyl-4-trifluorométhyl-1,4-dihydro-2H-3,1-benzoxazin-2-one (L-743 726 ou DMP-266) ; ou les NNRTIs de type quinoxaline, tels que le (2S)-7-fluoro-3,4-dihydro-2-éthyl-3-oxo-1 (2H)-quinoxalinecarboxylate d'isopropyle (HBY1293), est à administrer soit sous une forme pharmaceutique séparée, soit sous une forme pharmaceutique unique, avec ledit composé.

10. Composition pharmaceutique selon la revendication 6 pour son utilisation dans le traitement d'un patient diagnostiqué avec le SIDA, le para-SIDA (ARC) ; une lymphadénopathie généralisée progressive (PGL) ; le sarcome de Kaposi, le purpura thrombocytopénique ; des affections neurologiques liées au SIDA telles que la démence associée au SIDA, la sclérose en plaques ou la paraparésie tropicale ; des affections positives aux anticorps anti-VIH ou des affections positives au VIH.

11. Composition pharmaceutique selon la revendication 10, dans laquelle un agent thérapeutique supplémentaire choisi parmi la (1-alpha, 2-bêta, 3-alpha)-9-[2,3-bis(hydroxyméthyl)cyclobutyl]-guanine [(-)BHCG, SQ-34514] ; l'oxétanocine-G (3,4-bis-(hydroxyméthyl)-2-oxétanosyl]-guanine) ; les nucléosides acycliques, tels que l'acyclovir, le valaciclovir, le famciclovir, le ganciclovir ou le penciclovir ; les phosphonates de nucléosides acycliques, tels que la (S)-1-(3-hydroxy-2-phosphonyl-méthoxypropyl)cytosine (HPMPC) ; les inhibiteurs de ribonucléotide réductase tels que la 5-[(2-chloroanilino)thiocarbonyl]thiocarbonohydrazone de 2-acétylpyridine, la 3'-azido-3'-désoxythymidine ; d'autres 2',3'-didésoxynucléosides tels que la 2',3'-didésoxycytidine, la 2',3'-didésoxyadénosine, la 2',3'-didésoxyinosine, ou la 2',3'-didéshydrothymidine ; d'autres inhibiteurs d'aspartyl protéase, tels que l'indinavir, le ritonavir, le nelfinavir, ou l'ester de [3S-[3R*(1R*,2S*)]]-[3[[(4-aminophényl)sulfonyl]-(2-méthylpropyl)amino]-2-hydroxy-1-(phénylméthyl)-propyl]-tétrahydro-3-furanyle (amprénavir) ; les analogues de nucléosides oxathiolane, tels que la (-)-cis-1-(2-hydroxyméthyl)-1,3-oxathiolan-5-yl)-cytosine (lamivudine) ou la cis-1-(2-(hydroxyméthyl)-1,3-oxathiolan-5-yl)-5-fluorocytosine (FTC) ; la 3'-désoxy-3'-fluoro-thymidine ; la 5-chloro-2',3'-didésoxy-3'-fluorouridine ; le (-)-cis-4-[2-amino-6-(cyclopropylamino)-9H-purin-9-yl]-2-cyclo-pentène-1-méthanol ; la ribavirine ; la 9-[4-hydroxy-2-(hydroxyméthyl)but-1-yl]-guanine (H2G) ; les inhibiteurs de tat, tels que la 7-chloro-5-(2-pyrryl)-3H-1,4-benzodiazépin-2-(H) one (Ro5-3335) ou la 7-chloro-1,3-dihydro-5-(1H-pyrrol-2 yl)-3H-1,4-benzodiazépin-2-amine (Ro24-7429) ; les interférons, tels que l'interféron α ; les inhibiteurs d'excrétion rénale tels que le probénécide ; les inhibiteurs du transport des nucléosides tels que le dipyridamole ; la pentoxifylline ; la N-acétylcystéine (NAC) ; la procystéine ; l'α-trichosanthine ; l'acide phosphonoformique ; les immunomodulateurs, tels que l'interleukine II ou la thymosine ; les facteurs de stimulation des colonies granulocytaires/macrophages ; l'érythropoïétine ; les CD₄ solubles et leurs dérivés génétiquement modifiés ; les inhibiteurs de transcriptase inverse non nucléosidiques (NNRTIs), tels que la névirapine (BI-RG-587), le loviride (α-APA) ou la délavuridine (BHAP) ; les NNRTIs de type 1,4-dihydro-2H-3,1-benzoxazin-2-ones tels que la (-)-6-chloro-4-cyclopropyléthynyl-4-trifluorométhyl-1,4-dihydro-2H-3,1-benzoxazin-2-one (L-743,726 ou DMP-266) ; ou les NNRTIs de type quinoxaline, tels que le (2S)-7-fluoro-3,4-dihydro-2-éthyl-3-oxo-1 (2H)-quinoxalinecarboxylate d'isopropyle (HBY1293), est à administrer soit sous une forme pharmaceutique séparée, soit sous une forme pharmaceutique unique, avec ledit composé.
